# EUROPEAN PATENT SPECIFICATION

(11) **EP 2 191 016 B1**
(45) Date of publication and mention of the grant of the patent: **04.03.2015**
(21) Application number: 08788385.6
(22) Date of filing: 20.08.2008
(51) Int. Cl.: C12Q 1/68

(54) **ISOLATION OF PROTEIN FACTORS THAT ASSOCIATE DIRECTLY OR INDIRECTLY WITH NUCLEIC ACIDS**
ISOLIERUNG VON PROTEINFAKTOREN IN DIREKTER ODER INDIREKTER ASSOZIATION MIT NUKLEINSÄUREN
ISOLEMENT DE FACTEURS PROTÉIQUE QUI S'ASSOCIENT DIRECTEMENT OU INDIRECTEMENT À DES ACIDES NUCLÉIQUES NUCLEIC ACIDS

(30) Priority: 20.08.2007 US 935562 P
(43) Date of publication of application: 02.06.2010
(73) Proprietor: The General Hospital Corporation, Boston, MA 02114 (US)
(72) Inventor: KINGSTON, Robert, Boston, MA 2114 (US); DEJARDIN, Jerome, Boston, MA 2114 (US)
(74) Representative: Kehoe, Laura Ellen
(86) International application number: PCT/GB2008/002821
(87) International publication number: WO 2009/024781

(56) References cited:
- WO-A-2005/007814
- WO-A-2006/110314
- WO-A-2008/088839
- US-A1- 2005 170 346
- US-B1- 6 361 950
- MOROCHO A MICHAEL ET AL: "Biotin-labeled oligonucleotides with extraordinarily long tethering arms" METHODS IN MOLECULAR BIOLOGY, HUMANA PRESS INC., CLIFTON, NJ, US, vol. 288, 1 January 2005 (2005-01-01), pages 225-240, XP009107582 ISSN: 1064-3745 cited in the application
- MOROCHO A M ET AL: "Novel biotin phosphoramidites with super-long tethering arms" NUCLEOSIDES, NUCLEOTIDES AND NUCLEIC ACIDS, TAYLOR & FRANCIS, PHILADELPHIA, PA, vol. 22, no. 5-8, 1 January 2003 (2003-01-01), pages 1439-1441, XP009107662 ISSN: 1525-7770 cited in the application
- DUDIN G ET AL: "SORTING OF CHROMOSOMES BY MAGNETIC SEPARATION" HUMAN GENETICS, SPRINGER, BERLIN, DE, vol. 80, no. 2, 1 January 1988 (1988-01-01), pages 111-116, XP009070376 ISSN: 0340-6717
- AGALIOTI T ET AL: "Ordered recruitment of chromatin modifying and general transcription factors to the IFN-beta promoter." CELL 10 NOV 2000, vol. 103, no. 4, 10 November 2000 (2000-11-10), pages 667-678, XP002501253 ISSN: 0092-8674
- VESTER BIRTE ET AL: "LNA (locked nucleic acid): high-affinity targeting of complementary RNA and DNA." BIOCHEMISTRY 26 OCT 2004, vol. 43, no. 42, 26 October 2004 (2004-10-26), pages 13233-13241, XP002501252 ISSN: 0006-2960 cited in the application
- JIANG ET AL: "Magnetocapture of abalone transcription factor NF-kappaB: A new strategy for isolation and detection of NF-kappaB both in vitro and in vivo" JOURNAL OF BIOTECHNOLOGY, ELSEVIER SCIENCE PUBLISHERS, AMSTERDAM, NL, vol. 127, no. 3, 1 December 2006 (2006-12-01), pages 385-391, XP005787030 ISSN: 0168-1656
- TINGTING ET AL: "Subproteomic analysis of the cellular proteins associated with the 3' untranslated region of the hepatitis C virus genome in human liver cells" BIOCHEMICAL AND BIOPHYSICAL RESEARCH COMMUNICATIONS, ACADEMIC PRESS INC. ORLANDO, FL, US, vol. 347, no. 3, 1 September 2006 (2006-09-01), pages 683-691, XP005631867 ISSN: 0006-291X

## Description

### FIELD

The invention relates to assays for protein and polypeptide factors that associate with nucleic acid sequences, particularly genomic DNA and chromatin. In addition, novel chromatin associated factors identified by the assay are provided.

### BACKGROUND

Epigenetics concerns the transmission of information from a cell or multicellular organism to its descendants without that information being encoded in the nucleotide sequence of genes. Epigenetic mechanisms can operate through chemical modification of the DNA or through post translational modifications to proteins and polypeptides associated with the DNA.

In the genomes of eukaryotic cells, DNA is associated with protein complexes that assist in regulating gene expression, packaging of the DNA and controlling replication. The myriad of proteins that are associated with the genome contribute to what is termed chromatin: the nuclear material present in the nucleus of most eukaryotic cells. At various times in the cell cycle the level of packaging (or condensation) of the genomic DNA can vary between a lower packaged state such as during replication of the DNA (S Phase) to a more condensed state such as during cell division (M phase) where the genome is packaged into chromosomes. Highly expressed genes also tend to exist in a state of low packaging (so called euchromatic state), whereas silenced genes exist in a state of high packaging (so called heterochromatic state). The relative state of condensation, maintenance of this state and the transition between heterochromatin and euchromatin is believed to be mediated largely by a plurality of specialist proteins and polypeptide complexes.

At a fundamental level, the most 'open' or euchromatic form of chromatin comprises short sections of the genomic DNA wound around an octet of histone proteins, that together form a nucleosome. The nucleosomes are arrayed in series to form a beads-on-a-string formation. Interactions between adjacent nucleosomes allow the formation of more highly ordered chromatin structures. It is these interactions that can be mediated by enzymes that catalyse post-translational modifications of histones, or structural proteins that physically interact with and assist in anchoring the histones together.

Epigenetic controls over chromatin organisation and stability are essential for the normal and healthy functioning of a cell. Aberrant epigenetic modifications and a decrease in chromatin stability are often seen in senescent, apoptotic or diseased cells, particularly in cancer cells. It is of considerable importance to identify and characterise the multiple proteins and polypeptides that are capable of exhibiting epigenetic activities, as well as those factors that are capable of interacting with chromatin and chromatin associated proteins. It would also be of great value to identify and characterise novel chromatin associated factors, not least to facilitate a better understanding of chromatin biology as a whole.

Conventionally, isolation of proteins associated with chromatin has been achieved by performing a chromatin immunoprecipitation (ChIP). In a typical ChIP assay the chromatin binding proteins are crosslinked to DNA with formaldehyde in vivo. The chromatin is then sheared into small fragments and purified. The purified chromatin fragments are probed with antibodies specific to a known target chromatin binding protein so as to isolate the complex by immunoprecipitation. The precipitated chromatin is treated to reverse the cross-linking, thereby releasing the DNA for sequence analysis. Although it is possible to investigate the ancillary associated proteins pulled down by the cross-linking, the method is not restricted to one genomic region and is not optimised for this. Protocols for performing ChIP are disclosed in Nelson et al. (Nature Protocols (2006) 1:179 - 185) and Crane-Robinson et al. (Meth. Enzym. (1999) 304:533-547).

A significant drawback with ChIP based techniques is that for a given sequence, at least one specific protein associated with that sequence must be known already. Hence, is a need for a method of isolating protein factors that associate directly or indirectly with a specified target nucleic acid sequence. In effect, there is a need for a method of chromatin associated protein or polypeptide isolation that is nucleic acid sequence driven rather than antigen driven. Also, in ChIP a lack of immunoprecipitation does not necessarily reflect an absence of the tested factor, so there is always a rsk of false negative results with this technique.

WO-A-2008/088839 describes methods for isolating or purifying a biomolecule directly or indirectly bound to a region of interest of a nucleic acid, and methods for isolating or purifying a biomolecule that directly or indirectly binds to a region of interest of a nucleic acid. The methods and probes of WO-2008/088839 are optimised to function in prokaryotic systems, for example, a capture probe is used to identify a presumed polypeptide complex associated with a plasmid present in an *E.coli* cell lysate.

US-A-6361950 describes a solid phase assay system in which a radio-transmitter-receiver (a transponder) is attached to an oligonucleotide probe. The transponder typically has dimensions in the millimetre range. The spacer used to link the transponder to the oligonucleotide is an oligo (dT)10 sequence. The methods described in US-A-6361950 are incable of isolating polypeptides or proteins associated with a target DNA sequence, especially in chromatin, because the target DNA is denatured prior to hybridisation.

The present invention overcomes the deficiencies in the art by providing a novel method for isolating protein factors that associate directly or indirectly with a given target nucleic acid sequence. In particular the method of the invention overcomes the aforementioned problems with regard to isolating novel chromatin binding proteins and polypeptides.

### SUMMARY

In a first aspect the invention provides a method for isolating one or more polypeptides associated with a target nucleic acid sequence comprising:
(a) obtaining a sample that comprises a target nucleic acid sequence as well as one or more polypeptides that are associated with the target nucleic acid sequence;
(b) contacting the sample with at least one oligonucleotide probe that comprises a sequence that is complimentary to and capable of hybridising with at least a portion of the target nucleic acid sequence, wherein the oligonucleotide probe comprises at least one locked nucleic acid (LNA) nucleotide and wherein the oligonucleotide probe further comprises at least one affinity label and wherein the affinity label is conjugated to the oligonucleotide probe via a spacer group having a length of between 40 and 170 atoms; more suitably between 70 and 130 atoms; more typically between 90 and 120 atoms; suitably between 100 and 110 atoms;
(c) allowing the at least one oligonucleotide probe and the target nucleic acid sequence to hybridise with each other so as to form a probe-target hybrid;
(d) isolating the probe-target hybrid from the sample by immobilizing the probe-target hybrid through a molecule that binds to the at least one affinity label; and
(e) eluting the one or more polypeptides that are associated with the target nucleic acid sequence.

Since the nucleic sequence provides a universal means of discriminating a specific chromatin locus from others, the present invention has utilized a nucleic acid hybridization approach as the basis for a purification strategy. As such, when applied to chromatin the present method allows the isolation of specific chromatin regions and the subsequent identification of the proteins bound to those loci. The inventors have referred to the broadest aspect of the present invention when it is applied to use with chromatin as Proteomics of Intact Chromatin (PICh) because important protein-protein and protein-nucleic acid interactions are maintained during the purification procedure.

Typically the target nucleic acid sequence comprises eukaryotic or prokaryotic nucleic acid (e.g. RNA or DNA). Suitably the eukaryotic DNA is mammalian DNA and optionally the target nucleic acid sequence is comprised within chromatin. In this latter case, advantageously, the method of the invention is capable of isolating polypeptides that are associated with the DNA either individually or as part of multi-factorial complexes.

In a specific embodiment of the method of the invention, the one or more polypeptides associated with the target nucleic acid sequence are exposed to conditions that result in crosslinking of the one or more polypeptides. This crosslinking step occurs prior to the step of exposing the sample to the oligonucleotide probe (i.e. before step (b)), and the crosslinking is reversed prior to the step of eluting the one or more polypeptides (i.e. before step (e)). Optionally, the method of the invention further extends to analysing the eluted one or more polypeptides to determine their identity. Analysis can occur via any number of techniques known in the art, but typically high throughput proteomic analysis will include a mass spectrometry component, or an antibody microarray screen.

A second aspect of the invention provides an oligonucleotide probe comprising at least one group that conforms to general formula I, set out below:

A - [C]ₙ - X I

wherein A includes one or more affinity labels tethered to a nucleotide X by a spacer group C of n atoms in length, wherein n is between 90 and 170 atoms; A comprises a hapten or an immuno-tag; and wherein the nucleotide X is selected suitably from a ribonucleotide, a deoxyribonucelotide, a dideoxyribonucleotide and a locked ribonucleotide (LNA). Suitably the hapten is selected from biotin or an analogue thereof, such as desthiobiotin; digoxigenin; fluorescein; and or dinitrophenol. In a particular embodiment of the invention the oligonucleotide probe comprises first and second groups of the invention, wherein the first group comprises first affinity label A and the second group comprises a second affinity label A', and wherein A and A' are not the same.

A third aspect of the invention provides an oligonucleotide probe that conforms to general formula II, set out below:

B-[C]ₙ-Y II

wherein B is an affinity label that is tethered to oligonucleotide sequence Y via a spacer group C comprising a linear chain of n atoms, wherein n is between 90 and 170 atoms; the oligonucleotide sequence Y comprising at least 10 nucleotides of which no less than 10% are locked nucleic acid nucleotides; typically at least 25% of the nucleotides are locked nucleic acid nucleotides; optionally up to 100% of the nucleotides are locked nucleic acid nucleotides. Optionally, the carbon spacer group is linked to the 5' nucleotide of the oligonucleotide probe.

A fourth aspect of the invention provides an oligonucleotide probe that comprises a group that conforms to general formula III, set out below: 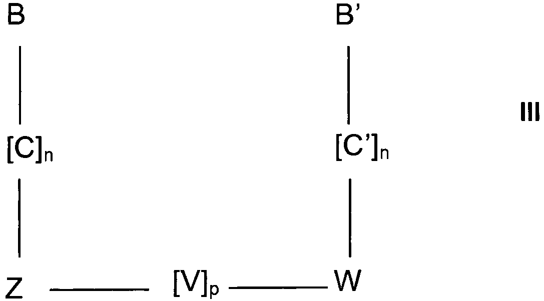 wherein B and B' include an affinity label that is the same or different tethered to respective nucleotides Z and W by spacer groups C and C' of n atoms in length, wherein n is between 90 and 170 atoms; and wherein the nucleotides Z and W are separated by an oligonucleotide chain V of p nucleotides in length, where p is between 0 and 40; the nucleotides Z, W and V being the same or different and selected suitably from a ribonucleotide, a deoxyribonucelotide, a dideoxyribonucleotide and a locked ribonucleotide (LNA) ), and at least one of Z and W is a LNA. Suitably the affinity label is selected from biotin or an analogue thereof, such as desthiobiotin; digoxigenin; fluorescein; and/or dinitrophenol. In a particular embodiment of the invention the oligonucleotide probe comprises the group of general formula III such that nucleotide Z represents the 5' nucleotide in the oligonucleotide probe.

Also described is a method of identifying a polypeptide that associates with a specific region of chromatin in the genome of a eukaryotic cell, comprising:
(a) identifying a target nucleic acid sequence present in the specific region of chromatin in the eukaryotic cell;
(b) constructing an oligonucleotide probe sequence that specifically hybridises with at least a portion of the DNA sequence located within the specific region;
(c) utilising the oligonucleotide probe in the method described above so as to isolate one or more polypeptides associated with the specific region of chromatin; and
(d) analysing the one or more isolated polypeptides so as to identify a polypeptide that associates with the specific region of chromatin.

The specific region of chromatin comprises one or more of: a telomere; a centromere; euchromatin; heterochromatin; intergenic regions; a gene; a repeat sequence; a heterologously inserted sequence; and an integrated viral genome. Typically the polypeptide that associates with a specific region of chromatin is further screened for enzymatic activity. Suitably, a polypeptide that associates with a specific region of chromatin is identified as a prospective drug target, especially if said target exhibits some form of enzymic activity.

Further described is a method of screening for a modulator of epigenetic activity comprising:
isolating a polypeptide that is identified as associating with a specific region of chromatin in the genome of a eukaryotic cell according to the methods described previously;
contacting the isolated polypeptide with one or more compounds from a library of compounds; and
identifying those compound(s) that bind to and modulate the activity of the isolated polypeptide as modulators of epigenetic activity.

The isolated polypeptide is selected from one of: Hint2; GMP synthase; Pri2; Fen1; USP7; TIF1-beta; Pin-1; Aurora kinase B; USP1 and MTA1. These specific targets are described in more detail below.

Yet further described is a method of characterising the biological activity of a polypeptide comprising the steps of:
isolating a polypeptide that is identified as associating with a specific region of chromatin in the genome of a eukaryotic cell according to the method described above;
obtaining the nucleic acid sequence for the polypeptide;
generating an antisense nucleic acid sequence that is complementary to all or a part of the nucleic acid sequence for the polypeptide;
introducing the antisense nucleic acid sequence into a eukaryotic cell so as to reduce expression of the polypeptide in the eukaryotic cell; and
analysing the phenotype of the eukaryotic cell so as to determine the biological activity of the polypeptide.

Suitably, the antisense nucleic acid sequence can comprise an siRNA oligonucleotide (or a precursor such as an shRNA). The eukaryotic cell can be a mammalian cell. Suitably, the introduction of the antisense nucleic acid sequence into a eukaryotic cell is to reduce expression of the polypeptide in the eukaryotic cell. This can be achieved by insertion of a heterologous sequence encoding the antisense nucleic acid sequence into the genome of the eukaryotic cell thereby generating a transgenic cell, via recombinant techniques known in the art (e.g. gene targeting/homologous recombination). Optionally, the eukaryotic cell is a pluripotent stem cell, such as an embryonic stem cell. Such a method allows for the production of transgenic non-human animals, such as 'knock out' mice.

### DRAWINGS

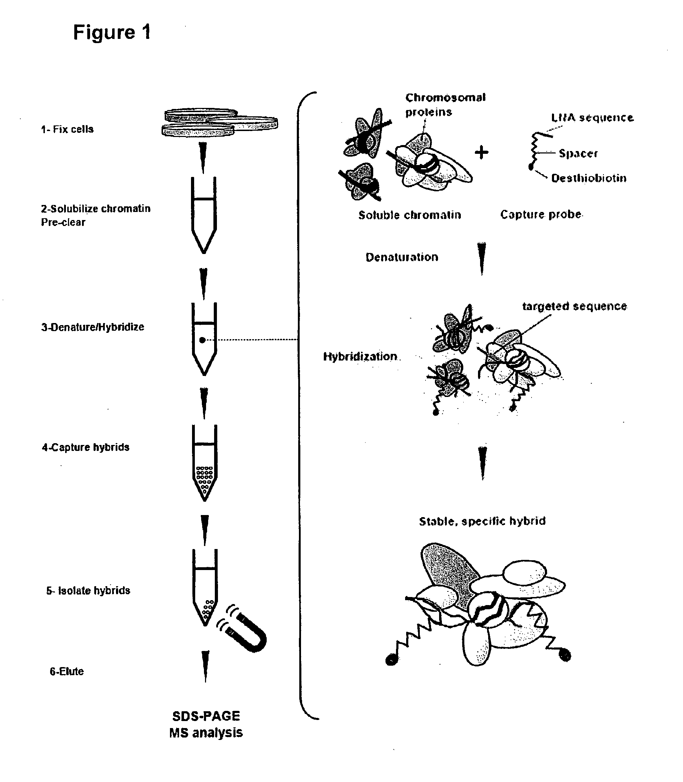

Figure 1 shows a graphical representation of one embodiment of the method of the invention.

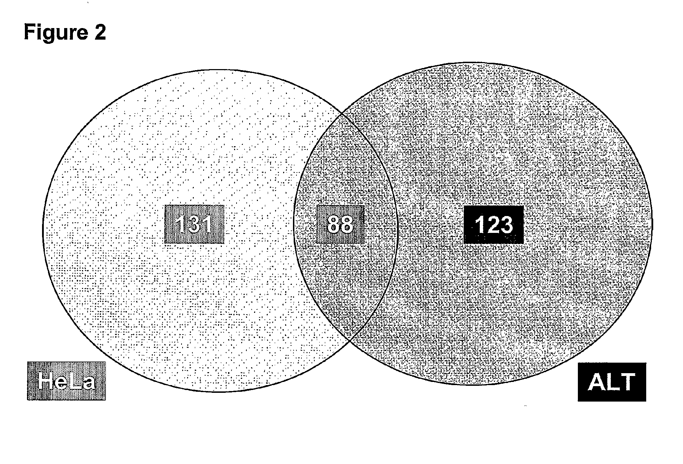

Figure 2 shows a graphical representation indicating the proportion of novel telomere chromatin associated polypeptide factors, identified according to the method of the invention, that are held in common between two different cancer cell types. The numbers in the circles indicate the number of novel telomere chromatin associated polypeptide factors identified in the specified cell types.

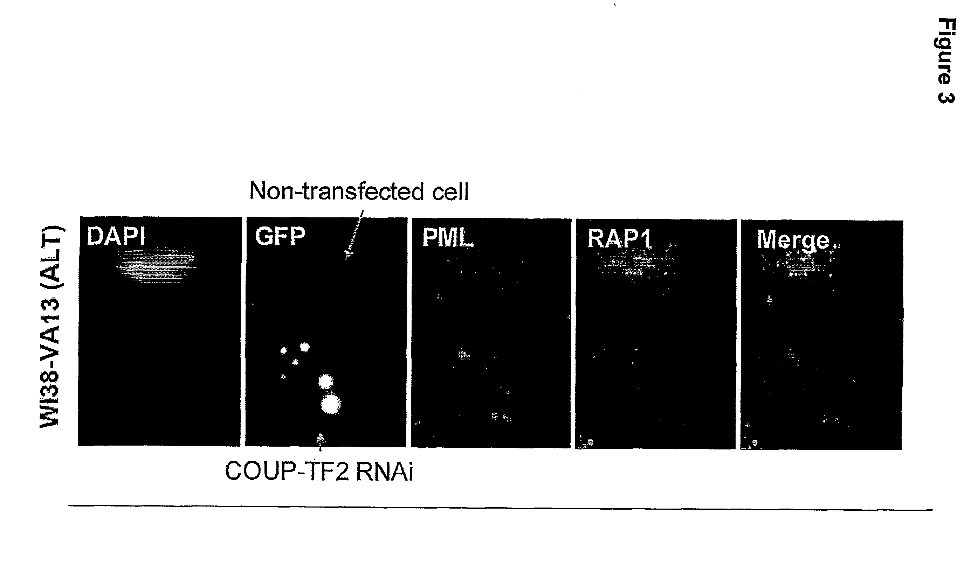

Figure 3 shows the results of an experiment to knock down the novel telomere associated chromatin factor COUP-TF2 in a transformed human fibroblast cell line. The PML antibody (PML) shows the location of PML bodies within the cells. The RAP1 antibody shows the location of telomeres in the cell. The merge image shows that telomeres (RAP1) do not localise to PML bodies (PML) in the presence of COUP-TF2 shRNA.

### DETAILED DESCRIPTION

Unless otherwise defined, all technical and scientific terms used herein have the same meaning as commonly understood by one of ordinary skill in the art to which this invention belongs. It will be understood that the present invention involves use of a range of conventional molecular biology techniques, which can be found in standard texts such as Sambrook et al. (Sambrook et al (2001) Molecular Cloning: A Laboratory Manual; CSHL Press, USA).

In setting forth the detailed description of the invention, a number of definitions are provided that will assist in the understanding of the invention.

The term "polypeptide" as used herein, refers to a polymer of amino acid residues joined by peptide bonds, whether produced naturally or in vitro by synthetic means. Polypeptides of less than approximately 12 amino acid residues in length are typically referred to as a "peptide". The term "polypeptide" as used herein denotes the product of a naturally occurring polypeptide, precursor form or proprotein. Polypeptides also undergo maturation or post-translational modification processes that may include, but are not limited to: glycosylation, proteolytic cleavage, lipidization, signal peptide cleavage, propeptide cleavage, phosphorylation, ubiquitylation, sumoylation, acetylation, methylation and such like. A "protein" is a macromolecule comprising one or more polypeptide chains.

A "polypeptide complex" as used herein, is intended to describe proteins and polypeptides that assemble together to form a unitary association of factors. The members of a polypeptide complex may interact with each other via non-covalent or covalent bonds. Typically members of a polypeptide complex will cooperate to enable binding either to DNA or to polypeptides and proteins already associated with or bound to DNA (i.e. chromatin). Chromatin associated polypeptide complexes may comprise a plurality of proteins and/or polypeptides which each serve to interact with other polypeptides that may be permanently associated with the complex or which may associate transiently, dependent upon cellular conditions and position within the cell cycle. Hence, particular polypeptide complexes may vary in their constituent members at different stages of development, in response to varying physiological conditions or as a factor of the cell cycle. By way of example, in animals, polypeptide complexes with known chromatin remodelling activities include Polycomb group gene silencing complexes as well as Trithorax group gene activating complexes.

The term "isolated", when applied to a nucleic acid or polypeptide sequence is a sequence that has been removed from its natural organism of origin. Typically, an isolated polypeptide or polynucleotide / nucleic acid molecule has been removed from the environment in which it was produced; although, it is not necessarily in a pure form. That is, an isolated polypeptide or polynucleotide is not necessarily 100% pure, but may be about 10%, 20%, 30%, 40%, 50%, 60%, 70%, 80% or 90% pure. A purified, isolated polypeptide or polynucleotide is advantageously at least 80% pure, and may be at least 90%, at least 95% or at least 98% pure (e.g. 99% pure). In the present context, the term "isolated" when applied to a polypeptide is intended to include the same polypeptide in alternative physical forms whether it is in the native form, denatured form, dimeric/multimeric, glycosylated, crystallised, or in derivatised forms. Advantageously, the nucleic acid molecules / polynucleotides / oligonucleotides (e.g. nucleic acid probes, RNAi molecules etc.), and polypeptides / peptides (e.g. antibodies or fragments thereof) of the invention are isolated; and more beneficially, purified.

Chromatin is the compacted structure of genomic DNA present in the nucleus of most eukaryotic cells. It comprises DNA and a plurality of DNA-binding proteins as well as certain RNAs. The term 'chromatin' derives from the readiness of this cellular material to hold stain with certain chemical dyes (chromaticity). Chromatin is primarily comprised of DNA associated with histone proteins that together form a basic nucleosomal structure. The nucleosome comprises an octet of histone proteins around which is wound a stretch of double stranded DNA 146 bp in length. Histones H2A, H2B, H3 and H4 are part of the nucleosome while histone H1 can act to link adjacent nucleosomes together into a higher order structure. Assembly into higher order structures allows for greater packing, or condensation of the DNA. Chromatin is often referred to as occurring in two main states, euchromatin and heterochromatin, corresponding to uncondensed actively transcribed DNA and condensed DNA respectively. Many further polypeptides and protein complexes interact with the nucleosome and the histones in order to mediate transition between the euchromatic and heterochromatic states. The identity and functional activity of many of these crucially important chromatin associated proteins and complexes is presently unknown. Epigenetics concerns the transmission of information from a cell or multicellular organism to its descendants without that information being encoded in the nucleotide sequence of genes. Epigenetic controls are typically established via chemical modification of the DNA or chromatin structure. Gene expression can be moderated, in some cases, via the covalent attachment of chemical groups to polypeptides that are associated with or that can bind to DNA. By way of example, methylation, sumoylation, phosphorylation, ubiquitylation and/or acetylation of histones can lead to activation or silencing of gene expression in the region of the genome where these epigenetic modifications have occurred. Epigenetic modifications can occur at different times in the normal development of an organism, and also during transformation of normal cells into cancerous cells. Such modifications often result in the silencing or activation of certain genes. In cancer, it is well documented that the majority of tumour cells display abnormal DNA epigenetic imprints (Feinberg AP & Vogelstein B, (1983) Nature 1 (5895):89-92).

The term "cancer" is used herein to denote a tissue or a cell located within a neoplasm or with properties associated with a neoplasm. Neoplasms typically possess characteristics that differentiate them from normal tissue and normal cells. Among such characteristics are included, but not limited to: a degree of anaplasia, changes in cell morphology, irregularity of shape, reduced cell adhesiveness, the ability to metastasise, increased levels of angiogenesis, increased cell invasiveness, reduced levels of cellular apoptosis and generally increased cell malignancy. Terms pertaining to and often synonymous with "cancer" include sarcoma, carcinoma, tumour, epithelioma, leukaemia, lymphoma, polyp, transformation, neoplasm and the like.

An embodiment of the present invention resides in the development of a method for identifying proteins, polypeptides and protein complexes that are associated with a particular target chromatin site, gene or stretch of nucleic acid, such as DNA. The method utilises a high specificity nucleic acid probe labelled with an affinity tag that allows for isolation of probe-target hybridised sequences. To determine whether a protein of interest is localized to a specific genomic region, the standard approach has been to combine immuno-staining and DNA fluorescent in situ hybridization (immuno-FISH) on fixed nuclei. However, previous attempts to retrieve target chromatin using conventional DNA capture/FISH probes and standard FISH reagents have always suffered from very low yields and high contamination from non-specific proteins. The method of the invention demonstrates an advantage of enabling the identification of any and all DNA and/or chromatin associated proteins at a specified target site without the need for prior knowledge of any of the proteins that may or may not be present at that site. Hence, the method of the invention also demonstrates considerable advantage over immuno-precipitation based techniques, such as ChIP, which rely on the presence of a known protein antibody target that is already bound to the DNA. Also, if the antibody is quantitatively precipitating a crosslinked antigen, which is rare, ChIP does not permit purification of a single loci but a mixture of loci that contain the protein of interest. The method of the invention also allows for changes in chromatin/ DNA associated protein complexes to be monitored under different cellular conditions as well.

A specific embodiment of the present disclosure is outlined in Figure 1. In brief, cells are fixed, the chromatin solubilized, a specific probe is hybridized to the chromatin, the hybridized chromatin is then captured on magnetic beads, the hybrids are eluted and the proteins identified. Extensive crosslinking with agents such as formaldehyde can be used to preserve protein-DNA and protein-protein interactions. Unlike strategies based upon antibody antigen affinity, nucleic acid hybridization is insensitive to the presence of ionic detergents, which allows the use of these detergents throughout to limit contamination. To increase the stability of the probe-chromatin interactions, Locked Nucleic Acid (LNA) containing oligonucleotides are used as probes because LNA residues have an altered backbone that favours base stacking thereby significantly increasing their melting temperature (Vester, B., and Wengel, J. (2004) Biochemistry 43, 13233-13241). To minimize the steric hindrance (which is detrimental for yields) observed upon immobilization of chromatin a very long spacer group is between the immobilization tag and the LNA probe. Suitable spacers include long chain aliphatic groups, or spacers can be synthesised from methoxyoxalamido and succinimido precursors such as those described in Morocho, A. M. et al (Methods Mol Biol (2005) 288, 225-240). Finally the co-elution of non-specific factors is limited by using desthiobiotin, a biotin analog with weaker affinity for avidin, permitting a competitive gentle elution using biotin.

In a further specific embodiment of the disclosure, protein complexes associated with telomere sequences in telomerase positive cancer cells and in an ALT (alternative lengthening of telomeres) cancer cell type were identified according to the method of the invention. In addition to the proteins and protein complexes expected to be present and associated to the telomere sequences, a surprising and unexpected number of additional polypeptides were identified. Some of the polypeptides included known proteins not previously expected to associate with chromatin, whereas other known chromatin associated proteins were newly identified as localising to telomeres. Hence, the invention further provides for identification and isolation of polypeptides with a novel chromatin association activity.

Accordingly, the present disclosure further resides in the provision of a subset of polypeptides that are newly identified as possessing chromatin association activity, and thus which potentially act as novel epigenetic factors. The invention facilitates the identification of further epigenetic factors and, importantly, the identification of novel epigenetic activity in known polypeptides.

In a specific embodiment, the present invention provides a method by which polypeptides and components of protein complexes associated with chromatin at a specified site in the genome can be characterised. It should be noted that the method of the invention is not limited to those polypeptides that are solely DNA binding, but includes associated polypeptides such as those with histone binding activity, for example.

In more detail, the method of the invention can comprise a first step, in which an affinity labelled nucleic acid probe sequence specifically hybridises to a preparation of sample chromosomal material comprising the chromatin target of interest. The probe is designed such that it hybridises with a specified target sequence in the DNA. The target sequence may suitably include a unique gene sequence, a repetitive sequence, or a sequence known to have a role in higher order structural formation. The probe sequence is introduced at a concentration and under stringent hybridisation conditions such that only the target sequence in the genomic DNA is bound.

The probe is labelled with one or more suitable affinity tags. Affinity tags may include immuno-tags or haptens. For example, one or more of the nucleotides contained within the probe sequence may be biotinylated (either with biotin or a suitable analogue thereof - e.g. desthiobiotin). Alternative affinity labels may include digoxigenin, dinitrophenol or fluorescein, as well as antigenic peptide 'tags' such as polyhistidine, FLAG, HA and Myc tags. For target sequences that are present in high copy number in the sample of interest, probes will typically comprise only a single type of affinity label. For targets of low concentration, such as single copy sequences in the genome of an organism, optionally the oligonucleotide probes of the invention may comprise more than one type of tag. By way of example, in an embodiment of the invention an oligonucleotide probe directed towards a single copy promoter region in a eukaryotic cell comprises LNA nucleotides labelled with both desthiobiotin and digoxigenin. The inclusion of more than one affinity tag in the probes of the invention can significantly increase the sensitivity of the process for low copy number targets.

Prior to the hybridisation step, the chromatin can be partially enzymatically digested in order to increase the resolution and to facilitate the next step of the method, which involves 'pull-down' of the probe-target sequence hybrid. Alternatively, the chromatin can be fragmented by physical methods such as ultrasonication, or by a combination of physical and enzymatic approaches.

The 'pull-down' step is facilitated by use of a binding moiety that engages the affinity tag and enables the hybridised sequences to be isolated. In case of a biotinylated probe sequence, isolation of the hybridised sequences can be effected in vitro by exposing the hybridised sequences to microbeads coated with streptavidin. In this way the hybridised sequences will bind to the beads and can be precipitated out of solution via a straightforward microcentrifugation step. Alternatively the microbeads may comprise a magnetic component allowing for immobilisation of the beads via exposure to a magnetic field (see Figure 1). Alternative isolation strategies include the immobilisation of the LNA containing probes on a solid substrate such as a microarray support or a dipstick. In this way the 'pull down' is facilitated by localisation to a specific area on a surface, which can then be suitably adapted so as to be suitable for use in later surface-enhanced laser desorption ionization time-of-flight mass spectrometry (SELDI-TOF-MS) analysis of the associated polypeptides.

The purified, or 'pulled-down' hybridised sequences comprise affinity labelled probe hybridised to the target sequence together with any associated chromatin polypeptides, proteins and polypeptide complexes that are bound to the target sequence. These associated chromatin polypeptides, proteins and polypeptide complexes can be isolated from the pulled-down material by standard protein precipitation steps and, if required, separated via electrophoretic (e.g. SDS-PAGE) or chromatographic techniques (e.g. HPLC).

The chromatin associated proteins and polypeptides can be analysed to determine their identity such as via high throughput polypeptide identification protocols suitably including the mass spectrometry based technique of peptide mass fingerprinting (PMF). Alternatively, qualitative changes in the composition of known chromatin associating complexes can be monitored using antibody array technologies that are directed to constituent members of the complexes of interest.

It will be appreciated that the method of the present disclosure is not limited to a specific type of genomic DNA and can be directed a virtually any target sequence in the genome in order to identify the associated protein and polypeptide profile. In addition, for any given sequence the method can be employed at different times in development, in the cell cycle or following exposure of the cell to external stimuli. As such, the method of the invention can allow for detailed profiling of the change in proteins and polypeptides associated with a specific target sequence to be monitored. Moreover, the method of the invention allows for the identification of novel DNA and chromatin associated proteins and polypeptide factors, many of which may be known proteins hitherto not considered as having epigenetic, DNA binding or chromatin-associating activities. In addition to providing information on the identity of proteins bound to a locus, the present invention provides information on the relative levels of abundant proteins bound to a given sequence in distinct cell types. By way of example, in the process of developing the methods of the invention for use on telomeres, the present inventors noticed fewer histones in telomeric preparations from HeLa S3 cells compared to telomeric preparations from HeLa 1.2.11 cells.

In a specific embodiment of the present disclosure, proteins and polypeptides associated with a particular telomeric repeat sequence in human cancer cells were identified. In vertebrates, the sequence (TTAGGG)ₙ is repeated in the telomeric regions of chromosomes (-5-15 kb in humans) and can represent around 0.01 to 0.03% of the total genome. This repeat sequence was selected because of its relatively high concentration and, thus, the relative ease of targeting with a nucleic acid probe. The nucleic acid probe included biotin-analogue labelled nucleotides and also incorporated locked nucleic acid (LNA) nucleotides which served to substantially increase the specificity of the probe for the target telomeric repeat sequence. A plurality of proteins and peptides were identified as associated with the target sequences, including known candidates such as members of the shelterin family of proteins which are believed to protect telomeres from damage. In addition to the known telomere associated proteins and polypeptides, a surprising number of polypeptides and proteins were identified that were not previously known to bind to or associate with telomeric regions of the chromosome. These novel telomere associated factors represent promising targets for further investigation, particularly as new drug discovery targets. Tables 1 to 3 set out the novel telomere associated chromatin factors identified according to the method of the present invention. It can be seen that several of the factors identified are known to be targets for post-translational modification.

In a further embodiments of the disclosure, lower abundance targets are considered. For example, in Drosophila melanogaster (fruit fly) the Fab-7 region represents a single copy chromatin fragment that is responsible for the maintenance of the transcriptional status of a homeotic gene named Abdominal-B. This fragment is named a Polycomb Response Element and recruits factors of the Polycomb and trithorax group of genes. Understanding the function of the Fab-7 site in Drosophila is critical to the understanding of the maintenance of epigenetic states in higher eukaryotes, as the basic chromatin regulation is typically conserved in higher organisms such as mouse and humans. The chromatin associated polypeptides and proteins comprised within complexes that bind to Fab-7 are believed to be central to improving the efficiency of stem cell therapies and may be subject to aberrant regulation in several cancers. Fab-7 is a more complex sequence to target compared to telomeres which are highly repetitive. As a result, Fab-7 requires the use of several distinct probes to cover the region in an overlapping or contiguous fashion, unlike for telomeres where a single probe can hybridize along the length of the targeted region.

A further embodiment of the disclosure targets the HIV promoter (located in the LTR of the pro-virus), which is integrated into the genome of an infected human T cell line. Understanding the regulation of this promoter is crucial in determining the aetiology and molecular mechanisms of HIV/AIDS. In particular, it is known that the pro-virus can remain silent for periods of time and it is critical to understand why reservoirs of infected cells escape therapies, because those cells contain so called 'dormant' virus. Determining the relevant epigenetic components and the state of the chromatin in the region of the HIV LTR promoter allows further insight into the mechanisms of disease and thereby identifies new candidate drug targets for HIV therapy. The invention allows for comparisons to be made between chromatin composition at the HIV LTR region in human T cell lines that model the two situations dormant or active, and this also provides important data that is useful in designing novel drug treatments for HIV/AIDS.

The probe nucleic acid sequences of the present invention comprises one or more locked nucleic acid (LNA) nucleotides. LNA nucleotides are bicyclic RNA analogues that contain a 2'-O, 4'-C methylene bridge in the ribose moiety. The methylene bridge restricts the flexibility of the ribofuranose ring and 'locks' the structure into a rigid C3-endo conformation. By altering the conformation of the nucleotide in this way, the resultant oligomer/polymer probe demonstrates enhanced hybridization performance and biostability. Oligomeric probe sequences that are less than approximately 15 nucleotides in length can suitably comprise up to 100% LNA. Typically, longer probe sequences comprise a mix of LNA and conventional RNA or DNA nucleotides. LNA containing probe sequences have demonstrated particular suitability for ISH applications (Silahtoroglu et al. (2004) Cytogenet Genome Res 107:32-37) and techniques for probe sequence design are described in Tolstrup et al. (Nucleic Acids Res. 2003 July 1; 31(13): 3758-3762).

The oligonucleotide probe, in specific embodiments, comprises at least one group that conforms to general formula I, set out below:

A- [C]ₙ- X

wherein A includes an affinity label tethered to the nucleotide X by a spacer group C of n atoms in length. Typically A comprises a hapten. In a specific embodiment A comprises biotin, or an analogue thereof such as a desthiobiotin molecule. The nucleotide X can be selected suitably from a ribonucleotide, a deoxyribonucelotide, a dideoxyribonucleotide and a locked ribonucleotide (LNA). The length of the spacer group is such that n is between 90 and 170 atoms; more suitably between about 75 and 130 atoms; more typically between about 90 and about 120 atoms; suitably between about 100 and about 110 atoms.

In a specific embodiment, the oligonucleotide probe conforms to general formula II, set out below:

B- [C]ₙ- Y

wherein B is a immuno-tag or a hapten that is tethered to an oligonucleotide sequence Y via a spacer group C comprising a linear chain of n atoms. The oligonucleotide sequence Y comprises at least 10 nucleotides of which no less than 10% are locked nucleic acid nucleotides; typically at least 25% of the nucleotides are locked nucleic acid nucleotides; optionally up to 100% of the nucleotides are locked nucleic acid nucleotides. In a specific embodiment of the invention, the oligonucleotide is 25 nucleotides in length and comprises around 50% LNA and 50% DNA nucleotides. The length of the spacer group C is such that n is between 90 and 170 atoms; more suitably between about 70 and 130 atoms; more typically between about 90 and about 120 atoms; suitably between about 100 and about 110 atoms. Optionally, the spacer group is linked to the 5' nucleotide of the oligonucleotide Y, with the 5' nucleotide optionally being a locked nucleic acid nucleotide.

A further specific embodment provides an oligonucleotide probe that comprises a group that conforms to general formula III, set out below: 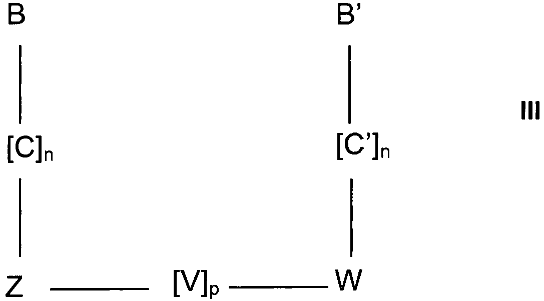 wherein B and B' include an affinity label that is the same or different tethered to respective nucleotides Z and W by spacer groups C and C' of n atoms in length wherein n is between 90 and 170 atoms; and wherein the nucleotides Z and W are separated by an oligonucleotide chain V of p nucleotides in length, where p is between 0 and 40, the nucleotides Z, W and V being selected suitably from a ribonucleotide, a deoxyribonucelotide, a dideoxyribonucleotide and a locked ribonucleotide (LNA), and at least one of Z, W and V is a LNA. In a particular embodiment of the invention the oligonucleotide probe comprises the group of general formula III such that nucleotide Z represents the 5' nucleotide in the oligonucleotide probe. The probes comprising the group of formula III are particularly suitable for targeting sequences in chromatin that have a low frequency of occurrence, such as single copy number sequences (e.g. particular genes). The separation between nucleotides Z and W is shown by the intervening oligonucleotide chain V, which is usually anything between 0 and 40 nucleotides in length, preferably between 1 and 30 nucleotides. Typically, nucleotides Z and W at least will be locked ribonucleotides. Suitably, spacer groups C and C' may be of the same or different lengths. In embodiments of the invention, the oligonucleotide probe may comprise one or more groups of formula III.

The probe nucleic acid sequences may be directed at any sequence that appears in, for example, genomic DNA. Advantageously, the invention is not limited to coding or non-coding sequences, nor is it restricted to use with euchromatic regions of the genome. Where the target sequence comprises a repeat sequence, such as a repeated telomeric sequence, a single probe species is often sufficient to effect pull down. In instances where a unique or less frequently repeated sequence is targeted it may be necessary to use a combination of two or more probes that bind to consecutive, overlapping or closely located regions of the target locus. In an example of the invention in use, purification of mouse pericentric heterochromatin is achieved using a combination of three probe oligonucleotides that hybridise with around to 25% of the target sequence. However, in order to optimise the pull down reaction the more target sequence covered by the probe(s), the greater the yield.

Identification of the protein and polypeptide factors found to be associated with the specified target sequence can be achieved through a number of routes. Typically the proteins and polypeptides are separated from the probe-target sequence hybrid by conventional protein extraction techniques. The proteins/polypeptides are then suitably purified broadly according to molecular weight. The separated proteins can be analysed by several methods to determine identity including western blotting. However, where the output of the method of the invention is expected to reveal one or more novel factors, mass spectrometry based techniques for protein identification are appropriately utilised. For instance, protein samples can be derived from SDS-PAGE and then optionally subjected to further chemical modification, such as reduction of disulfide bridges carboxymethylation of cysteine amino acid residues. The proteins/polypeptides are then cleaved into several fragments using a suitable proteolytic enzyme, such as trypsin. The proteolysis step is typically carried out overnight and the resulting cleaved peptides are then extracted with acetonitrile and dried under vacuum. The peptides are then dissolved in a small volume of distilled water and are ready for mass spectrometric analysis. Mass spectrometry can be performed on an aliquot of the purified peptide cleavage fragments via MALDI-TOF mass spectrometry. The output from the MALDI-TOF is then typically analysed in silico, using bioinformatics analytical techniques, and used to query online protein databases such as GenBank or SwissProt in order to identify and provide sequence information for the novel factor (for example, see Griffin et al. (1995) Rapid Commun. Mass Spectrom. 9(15):1546-51; and Courchesne & Patterson (1999) Methods Mol. Biol. 112:487-511).

Typically the mass spectrometry based techniques for polypeptide identification are referred to as peptide mass fingerprinting "PMF" after Pappin et al. (Curr Biol. (1993) Jun 1;3(6):327-32). PMF can identify proteins by matching the molecular masses of constituent fragments (peptide masses) to theoretical peptide masses generated for polypeptides in silico. The premise of PMF is that every polypeptide will possess a unique set of peptide fragments each with unique peptide masses. Identification of a given polypeptide is accomplished by matching the obtained peptide masses to the theoretical masses present in a PMF sequence database. PMF identification is optimised where there are several peptide fragments obtained from a given protein the mass of which is accurately known. Hence, MALDI-TOF mass spectrometry provides a particularly accurate means to determine the mass of each of these peptide fragments. Proteomic approaches can be used to determine the nature of protein complexes that are composed from the peptides and proteins identified via mass spectrometry (for example see Gingras et al. Nat Rev Mol Cell Biol. (2007) Aug;8(8):645-54).

By the term "modulator" it is meant a molecule (e.g. a chemical substance / entity) that effects a change in the activity of a target molecule (e.g. a gene, enzyme etc.). The change in activity is relative to the normal or baseline level of activity in the absence of the modulator, but otherwise under similar conditions, and it may represent an increase or a decrease in the normal / baseline activity. The modulator may be any molecule as described herein, for example a small molecule drug, an antibody or a nucleic acid. In the context of the present invention, the target is a novel chromatin associated factor that has been identified according to screening method of the invention. The modulation of chromatin-associated factor may be assessed by any means known to the person skilled in the art; for example, by identifying a change in the expression of genes regulated by the chromatin associated factor.

The present disclosure also relates to methods and compositions for the treatment of diseases associated with modified expression of one or more of the novel chromatin associated factors identified according to the method of the present invention.

Reagents for the inhibition of expression and/or biological activity of a specified chromatin associated factor include, but are not limited to, antisense nucleic acid molecules, siRNA (or shRNA), ribozymes, small molecules, and antibodies or the antigen binding portions thereof. For a review of nucleic acid-based technologies see, for example, Kurreck, J. (2003) "Antisense technologies - Improvement through novel chemical modifications", Eur. J. Biochem. 270: 1628-1644. The reagents for inhibition of the chromatin associated factor may affect expression and/or biological activity indirectly; for example, by acting on a factor that affects gene expression or that modifies or inhibits the biological activity of the novel chromatin associated factor. Advantageously, the reagent for use as an inhibitor of one of the novel chromatin associated factors identified herein acts directly on the chromatin associated factor, to affect gene expression at the mRNA level (e.g. transcription or mRNA stability), or the protein level (e.g. translation or biological activity).

Antisense nucleic acid sequences can be designed that are complementary to and will hybridise with a given mRNA *in-vivo.* Antisense nucleic acid sequences may be in the form of single stranded DNA or RNA molecules that hybridise to all or a part of the sequence of mRNA for the specified chromatin associated factor. Typically, an antisense molecule is at least 12 nucleotides in length and at least 90%, 93%, 95%, 98%, 99% or 100% complementary to the chosen target nucleotide sequence. Antisense oligonucleotides can be of any reasonable length, such as 12, 15, 18, 20, 30, 40, 50, 100, 200 or more nucleotides, having the advantageous above-mentioned complementarity to its corresponding target nucleotide sequence.

An antisense oligonucleotide may contain modified nucleotides (or nucleotide derivatives), for example, nucleotides that resemble the natural nucleotides, A, C, G, T and U, but which are chemically modified. Chemical modifications can be beneficial, for example, in: providing improved resistance to degradation by endogenous exo-and/or endonucleases, to increase the half-life of an oligonucleotide *in vivo;* enhancing the delivery of an oligonucleotide to a target cell or membrane; or increasing the bioavailability of an oligonucleotide. Typically, an antisense molecule contains a mixture of modified and natural nucleotides, and in particular, the 5' most and/or the 3' most nucleotides (e.g. the two outermost nucleotides at each end of the strand) may be modified to increase the half-life of the antisense molecule *in vivo.* In addition, or in the alternative, the backbone of an antisense molecule may be chemically modified, e.g. to increase resistance to degradation by nucleases. A typical backbone modification is the change of one or more phosphodiester bonds to a phosphorothioate bonds. An antisense molecule may suitably also comprise a 5' cap structure and/or a poly-A 3' tail, which act to increase the half-life of the antisense molecule in the presence of nucleases.

Antisense oligonucleotides can be used to inhibit expression of one or more chromatin associated factors identified according to the method of the present invention in target tissues and cells *in vivo.* Alternatively, such molecules may be used in an *ex vivo* treatment, or in an *in vitro* diagnostic test.

Requirements for the design and synthesis of antisense molecules against a specific target gene (via its corresponding RNA sequence), methods for introducing and expressing antisense molecules in a cell, and suitable means for modifying such antisense molecules are known to the person of skill in the art.

For example, antisense molecules for use in therapy may be administered to a patient directly at the site of a tumour (for example, by injection into the cell mass of the tumour), or they can be transcribed from a vector that is transfected into the tumour cells. Transfection of tumour cells with gene therapy vectors can be achieved, for example, using suitable liposomal delivery systems or viral vectors (Hughes, 2004, Surg. Oncol., 85(1): 28-35).

Another means of specifically down-regulating a target gene, such as a chromatin associated factor gene is to use RNA interference (RNAi). Naturally, RNAi is typically initiated by long double-stranded RNA molecules, which are processed by the Dicer enzyme into 21 to 23 nucleotides long dsRNAs having two-nucleotide overhangs at the 5' and 3' ends. The resultant short dsRNA molecules are known as small interfering RNAs (siRNAs). These short dsRNA molecules are then thought to be incorporated into the RNA-induced silencing complex (RISC), a protein-RNA complex, which acts as a guide for an endogenous nuclease to degrade the target RNA.

It has been shown that short (e.g. 19 to 23 bp) dsRNA molecules (siRNAs) can initiate RNAi, and that such molecules allow for the selective inactivation of gene function *in vivo,* for example, as described in Elbashir et al. (2001, Nature, 411: 494-498). Thus, this technique provides a means for the effective and specific targeting and degradation of mRNA encoding a chromatin associated factor in cells *in vivo.* Accordingly, the invention provides siRNA molecules and their use to specifically reduce or eliminate the expression in cells of one or more chromatin associated factors identified by the methods of the present invention.

As in the case of antisense and ribozyme technology, an siRNA or shRNA molecule for *in vivo* use advantageously contains one or more chemically modified nucleotides and/or one or more modified backbone linkages.

Pharmaceutical preparations of the disclosure are formulated to conform to regulatory standards and can be administered orally, intravenously, topically, or via other standard routes. The pharmaceutical preparations may be in the form of tablets, pills, lotions, gels, liquids, powders, suppositories, suspensions, liposomes, microparticles or other suitable formulations known in the art.

Thus, the present disclosure encompasses the use of molecules that can regulate or modulate activity or expression of the novel chromatin associated factors of the invention for treating disease. Typically diseases associated with aberrant activity or expression of chromatin-associated factors will include: cancer, premature aging, inflammatory disease, autoimmune disease, virally induced diseases and infections and infertility.

Novel chromatin associated factors (polypeptides or fragments thereof) identified by the methods of the invention can be recombinantly expressed individually or in combination to create transgenic cell lines and purified factors for use in drug screening. Cell lines over-expressing the chromatin associated factors or fragments thereof can be used, for example, in high-throughput screening methodologies against libraries of compounds (e.g. "small molecules"), antibodies or other biological agents. These screening assays may suitably be either cell-based assays, in which defined phenotypic changes are identified (analogous to calcium signalling in GPCR FLIPR screening), or can serve as the source of high levels of purified proteins for use in affinity-based screens such as radioligand binding and fluorescence polarisation.

In a specific embodiment, novel protein and polypeptide chromatin associated factors were identified as localising to ALT telomere sequences alone, HeLa telomere sequences alone or both (see tables 1-3). This demonstrates that the technique is capable of discriminating between different cellular environments. Alterations in telomere composition are believed to be significant in cancer progression and development, and the different protein sets identified in the assay as telomere-associated may lead to identification of targets and pathways that form useful therapeutic intervention points in different cancer types. Further review of a subset of these factors led to identification of proteins having enzymatic activity. It will be appreciated that factors possessing an enzymatic function can exhibit promising potential in drug discovery. A selection of the novel telomere chromatin associated factors are set out in more detail below together with a description of their known enzymatic activities. Clearly the present invention may provide additional information relating to the biological activities of these previously known proteins that renders them of even greater importance as potential drug targets.

### ALT cells only

The enzymes identified in this section may have potential as oncology targets in patients in whom the ALT telomere maintenance pathway has been activated and may provide a mechanism for attaining a degree of selectivity over non-ALT i.e. non-cancer cells in the same patient.
- Pri-2
- Hint2
- MTA1
- Pin-1
- LSD1

### PRI-2

This is the large subunit (58kDa, p58) of DNA primase, also known as PRIM2A in humans. It is believed that the protein is only enzymatically active in the presence of the smaller subunit, p49.

### Hint2

Hint2 is an adenosine monophosphate-lysine hydrolase, previously of uncertain function. Martin et al. (Gastroenterology (2006) 130:2179-2188) have reported a number of findings that suggest this is not a potential drug candidate in oncology because Hint2 levels are down-regulated in hepatocellular carcinomas and knockdown of Hint2 in HepG2 cells leads to decreased sensitivity to apoptotic stimuli. Overexpression of Hint2 in the same system has the converse effect, with overexpression of Hint2 in HepG2 xenografts leading to decreased tumour size. The data presented in this application suggest that telomere localisation of Hint2 may be significant in its cellular function.

### MTA1

MTA1 is a component of the nucleosome remodelling and deacetylating (NuRD) complex, and its expression is at least partly controlled at the transcriptional level by c-Myc (Zhang et al., PNAS, 2005, Vol 102, 13968-13973). It is also known to be an oestrogen receptor co-repressor (Martin et al., Breast Cancer Res Treat, 2006, Vol 95, 7-12). MTA1 acts in a complex with HDAC1 and HDAC2 (Yao & Yang, J Biol Chem, 2003, Vol 278, 42560-42568).

Expression of metastasis-associated gene 1 (MTA1) has been shown to be correlated with invasiveness of a number of tumours including thymoma (Sasaki et al., Cancer Lett 2001, Vol 174, 159-163) and prostate cancer (Hofer et al., Cancer Res, 2004, Vol 64, 825-829) and migration and invasion of immortalised keratinocytes (Mahoney et al., Oncogene, 2002 Vol 21, 2161-2170) and PANC-1 pancreatic carcinoma cells (Hofer et al., Br J Cancer, 2004, Vol 90, 455-462). In human MDA-MB-231 breast cancer cells with relatively high MTA1 expression, knockdown resulted in inhibition of growth and invasion (Nicolson et al., Clin Exp Metastasis 2003, Vol 20, 19-24). High expression levels of MTA1 have been reported to be associated with relatively poor prognosis in hepatocellular carcinoma (Hamatsu et al., Oncol Rep, 2003, Vol 10, 599-604). In node-negative breast tumours, high expression of MTA1 is associated with increased relapse risk, but such tumours may be sensitised to systemic therapies such as tamoxifen or anthracyclines (Martin et al., Breast Cancer Res Treat, 2006, Vol 95, 7-12).

In transgenic mice in which over-expression of MTA1 was targeted to the mammary gland, inappropriate mammary gland development and tumorigenesis were observed (Bagheri-Yarmand et al., Development, 2004, Vol 131, 3469-3479).

One mechanism by which MTA1 may influence tumour invasiveness is by enhancing both the transcriptional activity and protein stability of Hif-1 alpha. The protein stabilisation is via increased Hif-1 alpha deacetylation through increased HDAC1 expression (Yoo et al., EMBO J, 2006, Vol 25, 1231-1241). This would be consistent with the relationship between elevated MTA1 levels and intratumoural microvessel density in breast cancer (Jang et al., Cancer Sci 2006, Vol 97, 374-379).

### LSD1

LSD1 was the first histone demethylase to be identified (Shi et al., Cell, 2005, Vol 122, 654-658) and selectively removes monomethyl and dimethyl marks from histone H3 lysine 4 and lysine 9. LSD1 interacts with the androgen receptor to stimulate androgen receptor-dependent transcription (Metzger et al., Nature, 2005, Vol 437, 436-439). LSD1 levels are up-regulated in high-risk prostate tumours (Kahl et al., Cancer Res, 2006, Vol 66, 11341-11347). LSD1 is required for cell proliferation and deficiency of the protein leads to partial cell cycle arrest and sensitisation to growth suppression by external stimuli (Scoumanne & Chen, J Biol Chem, 2007, Vol 282, 15471-15475). These features all make LSD1 an attractive oncology target. The reason for its hitherto unreported association with the telomere represents a fruitful area for investigation.

### Pin-1

Pin-1 is a peptidyl prolyl isomerase involved in the isomerisation of numerous oncogenic and cell-signalling pathways (reviewed in He et al., Lung Cancer, 2007, Vol 56, 51-58). Over-expression of Pin-1 has been reported in a number of cancers including non-small cell lung cancer (He et al., Lung Cancer, 2007, Vol 56, 51-58), and hepatocellular carcinoma where it may act synergistically with the hepatitis B-encoded HBx oncogene (Pang et al., Gastroenterology, 2007, Vol 132, 1088-1103). Activation of Stat-3 by Pin-1 leads to increased epithelial to mesenchymal transition in breast cancer cells (Lufei et al., Oncogene, 2007, Jun 11, e-publication ahead of print). In mouse models dual ablation of Pin-1 and p53 leads to accelerated thymic hyperplasia (Takahashi et al., Oncogene, 2007, Vol 26, 3835-3845).

Pin-1 is therefore an attractive oncology target for drug discovery. However, Pin-1 activity appears to play an important protective role in the amyloidogenic pathways implicated in Alzheimer's disease (see for example, Nowotny et al., Neurosci Lett, 2007, Vol 419, 15-17; Wang et al., J Alzheimers Dis, 2007, Vol 11, 13-23; Balastik et al., Biochim Biophys Acta, 2007, Vol 1772, 422-429). It may therefore be necessary to develop Pin-1 inhibitors that do not cross the blood-brain barrier.

### HeLa cells only

Some telomere-associated proteins were detected only in HeLa cells and not in the ALT-positive cell line. This may imply that the use of the ALT telomere maintenance system not only induces localisation of certain proteins to the telomere, it also prevents association of others (HeLa telomeres are maintained by telomerase, and as such, one can expect telomerase associated proteins, not found in ALT, such as GAR1 or BAT1). This may again provide a mechanism for patient stratification for therapy in cancer.
- Aurora B
- USP1

### Aurora B

Aurora kinase B is a chromosomal passenger protein that forms a complex with inner centromere protein and survivin (reviewed in Sistayanarain et al., Anticancer Res 2006, Vol 26, 3585-3593). Its association with the telomeres in HeLa cells as detected during our screen is entirely unexpected. Aberrant Aurora B activity is believed to be significant in a number of cancers including lung (Hayama et al., Cancer Res, 2007, Vol 67, 4113-4122; Vischioni et al., Mol Cancer Ther 2006, Vol 5, 2905-2013); oral squamous cell carcinoma (Qi et al., Virchows Arch, 2007, Vol 450, 297-302); breast cancer (Tchatchou et al., Cancer Lett, 2007, Vol 247, 266-272); prostate cancer (Lee et al., Cancer Res, 2006, Vol 66, 4996-5002); glioblastoma (Zeng et al., J Clin Pathol, 2007, Vol 60, 218-221) and mesothelioma (Lopez-Rios et al., Cancer Res, 2006, Vol 66, 2670-2679). In an animal model of renal tumours, Aurora B expression is oestrogen-responsive (Hontz et al., Cancer Res, 2007, Vol 67, 2957-2963).

Small molecule inhibition of Aurora B radio-sensitises mesothelioma cells (Kim et al., Int J Radiat Biol Phys, 2007, Vol 67, 1519-1525) and the inhibitors are themselves antiproliferative (Girdler et al., J Cell Sci, 2006, Vol 119, 3664-3675).

### USP1

USP1 is a cysteine protease which deubiquitinates mono-ubiquitinylated PCNA. Mono-ubiquitinylated PCNA is required for the translesion synthesis of damaged DNA. Inhibition of USP1 leads to an accumulation of this form of the protein (Huang et al., Nat Cell Biol 2006, Vol 8, 339-347).

### Both ALT and HeLa cells

Certain proteins were found to be associated with the telomeres of both ALT and non-ALT cells. This suggests that in addition to certain specific proteins for these cellular conditions, there is a smaller group of proteins that associates with telomeres in both patho-physiological settings. Selection of proteins not previously known to be associated with telomeres may indicate new therapeutic intervention points that could not previously have been anticipated.
- GMP synthase
- Fen1
- USP7
- PRMT1
- Transcription intermediary factor 1 beta

### GMP Synthase

GMP synthase is an amidopeptidase that catalyses the amination of xanthosine 5'-monophosphate to form GMP in the presence of glutamine and ATP (Nakamura et al., JBC, 1995, Vol 270, 23450-23455. This makes it a key enzyme in the de novo synthesis of guanine nucleotides. Given the requirement for nucleotides in rapidly dividing cells enzymes in the nucleotide synthesis pathways can be promising targets for drug discovery. The best known and clinically most useful example is 5-FU, the thymidylate synthase inhibitor. More recently GMP synthase has been shown to interact with Usp7 (see below) to stimulate histone H2B deubiquitinylation (van der Knaap et al., Mol Cell, 2005, Vol 17, 695-707) in Drosophila.

### Fen1

Fen1 is the structure-specific flap endonuclease involved in long-patch base excision repair involving multi-nucleotide DNA synthesis (Prasad et al., J Biol Chem, 2000, Vol 275, 4460-4466), and in Okazaki fragment renewal. The enzyme cleaves substrates with unannealed 5' ends and its activity is increased 5-50 fold by association with PCNA (Tom et al., J Biol Chem, 2000, Vol 275, 10498-10505).

Knockout of Fen1 is embryonically lethal in mice, whereas double heterozygotes for Fen1 and Apc1 develop increased numbers of adenocarcinomas and decreased survival (Kucherlapati et al., PNAS, 2002, Vol 99, 9924-9929). Fen1 (-/-) blastocytes are very sensitive to gamma irradiation, showing extensive apoptosis (Larsen et al., Mol Cell Biol, 2003, Vol 23, 5346-5353). In the chicken DT40 cell line, the cells are viable in the absence of Fen1 but have a slow growth phenotype, probably due to a high rate of cell death, and are hypersensitive to methylmethane sulfonate and hydrogen peroxide but not to UV irradiation, X-rays and etoposide (Matsuzaki et al., Nucleic Acids Res, 2002, Vol 30, 3273-3277). In normal mouse embryonic fibroblasts, Fen1 levels increase in response to UV irradiation, and this effect is p53-dependent (Christmann et al., Oncogene, 2005, Vol 24, 8403-8413).

Fen1 levels have been reported to be upregulated in lung cancer (Sato et al., 2003, Oncogene, Vol 22, 7243-7246) and the gene is also upregulated in 5-FU-resistant HCT116 colon cancer cell lines (de Angelis et al., Int J Oncology, 2004, Vol 24, 1279-1288). Mutations of Fen1 in human cancers have been identified and transgenic mice harbouring these mutations were predisposed to autoimmunity, chronic inflammation and cancers (Zheng et al., Nat Med, 2007 June 24, Epub ahead of print).

### USP7

USP7 cysteine protease is a component of the PML (promyelocytic leukaemia) nuclear bodies present in most mammalian cells (Muratani et al., Nat Cell Biol, 2002, Vol 4, 106-110). It is a target of at least two viral proteins - ICP0 of HSV type I and EBNA1 of EBV (Holowaty et al., J Biol Chem, 2003, Vol 278, 47753-47761). EBNA1 binds to the same region of USP7 as p53, whereas ICP0 binds to a different region.

Interaction of USP7 with ICP0 protects the viral protein from auto-ubiquitinylation and thereby stabilises it (Canning et al., J Biol Chem 2004, Vol 279, 38160-38168). ICP0 is an immediate-early regulatory protein that stimulates lytic infection and reactivation from latency.

Early reports claimed that USP7 has deubiquitination activity and is a strong stabiliser of p53, even in the presence of excess Mdm2 (Li et al., Nature, 2002, Vol 416, 648-653). However, later work from the same group suggested a more dynamically-determined effect, whereby partial reduction of endogenous USP7 levels by RNAi destabilised p53, but near complete ablation led to p53 stabilisation. The authors claim this is because USP7 is also required for Mdm2 stabilisation, so that in USP7-ablated cells, self-ubiquitinated Mdm2 becomes very unstable leading to p53 stabilisation and activation (Li et al., Mol Cell, 2004, Vol 13, 879-886).

Work from Cummins and Vogelstein supported the assertion that USP7's most important interaction is to deubiquitinate and thus stabilise Mdm2, leading to decreased levels of p53, by showing that in USP7-deficient mice Mdm2 becomes unstable and p53 levels are elevated (Cummins & Vogelstein, CellCycle, 2004, Vol 3, 689-692). USP7 is also involved in a further complex levels of control of p53 via the Mdm2 regulating/regulated protein Mdmx (Meulmeester et al., Mol Cell, 2005, Vol 18, 565-576). Further levels of regulatory complexity are added by the effects of the ATM protein kinase, which by phosphorylating Mdm2 and Mdmx, weakens their affinity for USP7 (Meulmeester et al., Cell Cycle, 2005, Vol 4, 1166-1170).

It has recently been speculated that one of the major effects of USP7 is to generate apoptotically active non-ubiquitinylated p53 at the mitochondria (Marchenko et al., EMBO J. 2007, Vol 26, 923-934).

USP7 appears to have other cellular ubiquitinated targets. In a complex with GMP synthase (see above) it catalyses the deubiquitinylation of histone H2B, but not H2A (van der Knaap et al., Mol Cell, 2005, Vol 17, 695-707), at least in Drosophila embryos. It also deubiquitinates FOXO, negatively regulating its transcriptional activity (van der Horst et al., Nat Cell Biol, 2006, Vol 8, 1064-1073) and Chfr, a checkpoint protein (Oh et al., Biochem Biophys Res Commun 2007, Vol 357, 615-619).

Perhaps surprisingly, levels of USP7 were reduced in 59/131 patients with NSCLC, and this was associated with reduced p53 expression (Masuya et al., J Pathol, 2006, Vol 208, 724-732). Targeting USP7 may be a viable therapeutic approach for haematopoietic tumours.

### PRMT1

PRMT1 is an arginine methyltransferase whose targets include histone H4, GRIP1 and PPARγ co-activator-1α (reviewed in Lee et al., Mol Endocrinol, 2007, Vol 21, 1381-1393), DNA polymerase beta (EI-Andaloussi et al., FASEB J, 2007, Vol 21, 26-34) and RIP140 (Mostaqul Huq et al., EMBO J, 2006, Vol 25, 5094-5104). PRMT1 is regulated by hCAF1 (Robin-Lespinasse et al., J Cell Sci, 2007, Vol 120, 638-647). Histone H4 methylation is an early and key event in oestrogen receptor-mediated gene regulation (Wagner et al., J Biol Chem, 2006, Vol 281, 27242-27250). Induction of interferon α/β-mediated gene transcription is also critically determined by the activity of PRMT1, in this case operating via Stat1 (Mowan et al., Cell, 2001 Vol 104, 731-741). Inhibition of PRMT1 activity has the potential to affect gene expression on a large scale.

### Transcription intermediary factor - 1 beta

TIF-1β, also known as TRIM28 has been identified as a component of transcriptional regulatory complexes (Friedman et al., Genes Dev 1996, Vol 10, 2067-2078; Moosmann et al., Nucleic Acids Res 1996 Vol 24, 4859-4867). Although not believed to bind DNA itself, TRIM28 is known to interact with a wide variety of nuclear factors including KRAB repressors (Kim et al., Proc Natl Acad Sci USA, 1996 Vol 93, 15299-15304), the histone binding protein HP1, SETDB1 (ESET), and MDM2 a key regulatory factor of p53 (Wang et al., Embo J 2005 Vol 24 3279-3290).

TRIM28 is considered to be a member of several larger multi-factor complexes that typically function in repression of gene expression and stabilisation of heterochromatin (Sripathy et al., Mol. Cell. Biol. 2006 Vol 26, 8623-8638). Sequence analysis of TRIM28 suggests the presence of several recognisable domains including N-terminal RING and coiled-coil domains that are believed to be involved in cofactor recognition and interaction (Peng et al., J. Mol. Biol. 2000 Vol 295, 1139-62), as well as C terminal PHD and bromo domains that are believed to interact with transcriptional repressors such as HP1 and SETDB1.

It is apparent, therefore, that the information derived from the method of the present invention allows for the accurate identification of a chromatin activity for these factors to the in the cell. By providing a cellular context for these diverse factors, as well as information on potential co-factors, the present invention allows for a more focussed approach to drug discovery and target selection. The identification of the proteins that interact with genomic regions of interest is also critical to the understanding of genome biology. These questions have previously been studied using genetics, biochemical characterization of soluble complexes, structural studies, chromatin immunoprecipitation, and cell biology. By establishing the 'chromatin formula' of factors bound at specific loci, the methods of the present invention significantly advance the characterization of chromosomes. By way of exemplification, in using the present methods to study telomeres, the present inventors identified most previously known factors in a single experiment, and found a plurality of new proteins that are now considered to be relevant to telomere biology. The surprisingly high number of proteins found at each class of telomeres (-200) underscores the diversity of events occurring at this locus. Clearly, the methods of the present invention have the ability to identify factors that would be difficult to uncover using genetics because they either play vital roles elsewhere or are redundant (e.g. orphan receptors).

The invention is further illustrated by the following non-limiting examples.

### EXAMPLES

The present inventors have devised a technique that isolates chromatin with associated bound factors in sufficient purity to allow identification of the proteins and polypeptides at the targeted locus. By way of exemplification, proteins associated with medium repeat elements present in mammalian chromatin have been purified and identified directly using mass spectrometry. Applying this technology to human telomeres, 92% of all proteins previously shown to bind telomeres were identified, including proteins with low level expression. In the following examples HeLa telomeres were compared with ALT telomeres, and a plurality of factors were identified that bind specifically to ALT telomeres. By this, it is inferred that via a change in the sequence of those telomeres different proteins will bind to the chromatin

The technique can be adapted to a variety of experimental situations, including viral integration sites, specific knockout cells, and post-drug treatment changes to name but a few. The method of the invention advantageously allows identification of changes in protein complexes associated with a target chromatin site, without requiring prior knowledge of any of the proteins that may already be associated with that target site.

### Example 1

### 1. Preparation of the chromatin template:

The starting cells were HeLa S3 cells (a human cancer cell line). From suspension, cells in spinner flasks (20 litres culture at a density of 0.5-1 10⁶ cells/ml) were pelleted by centrifugation at 2500g for 10 minutes at room temperature, and then immediately resuspended in cross-linking solution (200ml for 10¹⁰ cells; crosslinking solution: 3% formaldehyde into 1X PBS (from formaldehyde 37%, methanol stabilized solution)). For adherent cells, the media was first discarded and crosslinking solution is immediately added to the plates (10ml/15cm plate). Cells were incubated in crosslinking solution for 30 minutes at room temperature.

Cells in suspension were spun down at 3200g for 10 minutes at 4°C and the supernatant discarded. The material was aliquoted into 4 Falcon tubes (usually 8 ml pellet/tube). For adherent cells the crosslinking solution was discarded and the plates washed twice with 1X PBS solution (standard phosphate buffered saline solution supplemented with 1mM PMSF). A further 3ml of cell scrapping solution (1XPBS; 0.05% Tween-20) was added per plate and the cells were pooled into Falcon tubes on ice.

The cells were then washed four times in PBS by resuspending the cell pellet in 1XPBS solution bringing the volume to 50 ml/tube, then spinning down at 3200g for 10 minutes at 4°C. The supernatant was discarded each time and the final washed pellet was resuspended in sucrose solution (bringing the volume to 50 ml/tube). The solution was spun down at 3200g for 10 minutes at 4°C, the supernatant discarded and the pellet brought up to a volume of 40ml with sucrose solution.

The mixture was transferred to a 40ml Dounce homogenizer on ice and dounced 20 times with a tightly fitting pestle. The dounced mixture was transferred to a fresh Falcon tube and spun down at 3200g for 10 minutes at 4°C. The supernatant was discarded and the pellet resuspended in 50ml of glycerol buffer. This step was repeated once and the pellet resuspended in an equal volume of glycerol buffer to that of the pellet (that is, if the pellet volume is 7.5 ml, 7.5ml of glycerol buffer is added). The preparation can be aliquoted into 1.5ml volumes (that is ∼0.5 109 cell equivalent per 1.5ml Eppendorf tube) and is then either snap frozen liquid nitrogen and stored at -80°C or taken to the next step for the target pull-down. For the purposes of the telomere sequence pull down 3.10⁹ cell equivalent is used, equivalent to six 1.5ml Eppendorf tubes of the preparation.

The above chromatin preparation protocol is suitable for use with eukaryotic cells, particularly mammalian cells. However, it will be appreciated that the methods of the present invention are not restricted to identification of protein and polypeptide factors that are solely associated with eukaryotic genomic DNA and chromatin. Protocols for purification of nucleic acid plus associated factors are also known in prokaryotes as well as viruses.

### 2. Optimising the chromatin template for the hybridisation step:

The next step is described in reference to a single 1.5ml aliquot of the chromatin template preparation as the starting point.

Using a table top centrifuge, the chromatin template preparation was spun down at 2000g for 2 minutes at room temperature. The pellet was resuspended in 1.5ml of 1X PBS-Triton solution (1XPBS; 0.5% Triton-X100) and 15□µl of Concentrated RNAseA (Qiagen 100mg/ml) was added. The solution was incubated for 60 minutes at room temperature with shaking (using an Eppendorf Thermomixer at 1400rpm) then for a further at 4°C (without shaking) for 12-16 hours. This RNAse step can be omitted if the intention of the protocol is to purify ribonucleoprotein complexes that are or are not chromatin associated (e.g. Xist coated chromatin domains on the inactive X chromosome).

The RNAse treated (or untreated if necessary) mixture is pooled with the other treated aliquots in a 50ml Falcon tube, bringing the volume to 50ml with 1X PBS. The pooled mixture (now corresponding to the starting 6 aliquots) is spun down at 3200g for 10 minutes at 4°C and the supernatant is discarded. The washing step can be repeated up to 6 times to dilute out traces of any RNAseA that may be present. Then the pellet was resuspended in 50ml of fresh LB3JD buffer (10 mM HEPES-NaOH pH 7.9; 100 mM NaCl; 2 mM EDTA pH 8; 1 mM EGTA pH 8; 0.2% SDS; 0.1% Sarkosyl; make fresh, keep at room temperature and add PMSF to 1mM final concentration) at room temperature. The solution was spun down at 3200g for 10 minutes at 4°C, to produce a resulting pellet with typical volume of ∼4.5 ml. To the pellet was added 2.5ml of LB3JD buffer to resuspend. The resultant viscous solution was divided into two 3.5ml aliquots in Falcon 15ml tubes. The solutions were sonicated on ice in a 4°C cold room (Misonix 3000 sonicator with a micro-tip) The sonication parameters were: power setting 7, 15 seconds constant pulse, 45 seconds pause with a 7 minutes total process time. These conditions will usually give chromatin fragments that are about 2-4kb in length which is a suitable size for the subsequent hybridisation and pull down steps. The sonicated solution was divided into 1 ml aliquots and spun down at 16000g for 15 minutes at room temperature. The supernatants were pooled into a 15 ml Falcon tube. 400 µl aliquots of the sonicated chromatin solution were run each on S-400-HR gel filtration spin columns (Microspin™, GE Healthcare) at 800g for 2 minutes so as to reduce the salt concentration in the solution.

The eluates from the columns were pooled in a 15ml Falcon tube and incubated at 58°C for 5 minutes, which helps to unmask any endogenously biotinylated proteins present in the soluble chromatin preparation. The pooled solution was cooled to room temperature. 0.5ml of Ultralink Streptavidin bead slurry was equilibrated in, and washed twice with 10 ml of LB3JDLS buffer (10 mM HEPES-NaOH pH 7.9; 30 mM NaCl; 2 mM EDTA pH 8;1 mM EGTA pH 8; 0.2% SDS; 0.1% Sarkosyl; make fresh, keep at room temperature and add PMSF to 1 mM final concentration) and spun down at 3200g for 2 minutes. The supernatant was discarded leaving a slurry pellet of around 0.5ml volume. The washed streptavidin beads were added to the pooled soluble chromatin solution and the mixture was incubated for 2 hours at room temperature on a nutator. The solution was spun down at 3200g for 10 minutes at room temperature to remove most of the endogenously biotinylated proteins, and thereby reducing background in the later steps. The supernatant was saved and contains the 'cleared' soluble chromatin solution.

The chromatin solution was now ready for hybridization. It had the following spectrophotometric characteristics:
- DNA Concentration (O.D.260): 2-2.5 mg/ml
- O.D.260/O.D.280 = 1.30-1.45.

Higher values for the O.D. ratio would mean that the initial RNAseA treatment was not fully effective and that the purified material might be contaminated by non chromatin RNA-protein complexes. These values were obtained when LB3JDLS buffer was used as the 'blank' solution.

The solubilised and cleared chromatin solution can be stored at 4°C.

### 3. Hybridization and target sequence capture (pull down)

The solubilised chromatin samples obtained in the previous step were spun down for 15 minutes at 16 000g at room temperature. The aliquots were pooled together to give about 5ml of chromatin sample, to which was added 20% SDS to 0.02% final concentration (1/1000 of volume of chromatin sample).

To the chromatin solution was added 30 µl of the desired oligonucleotide probe sequence (from 100µM stock solution, see part 4 for details of the probe). A control reaction containing a probe having a scrambled or randomised sequence was also run in parallel.

The hybridisation reaction was divided into approximately 34 X 150 µl aliquots which were placed in a thermocycler for the hybridisation step (HYBAID Px2, Thermo Fisher).

The hybridisation program was as follows:
25°C for 3 minutes
70°C for 6 minutes
38°C for 60 minutes
60°C for 2 minutes
38°C for 60 minutes
60°C for 2 minutes
38°C for 120 minutes
25°C final temperature

The hybridised samples were pooled back into 1.5ml Eppendorf tubes and spun down at 16000g for 15 minutes at room temperature so as to remove any precipitate that may have formed during the hybridization step.

2.4 ml of MyONE C1 magnetic streptavidin beads (Invitrogen) were placed in a 15ml Falcon tube, to which 7.6 ml of LB3JD buffer was added to equilibrate them. The beads were immobilized on the magnetic stand and the supernatant was discarded. The beads were washed in 10 ml of LB3JD buffer, immobilized again and resuspended into 2.4ml total volume of LB3JD buffer.

The supernatant from the spun down hybridised chromatin samples was transferred to two 15ml Falcon tubes. To each tube was added 3.5 ml of milliQ water and then 1.2ml of C1 magnetic bead solution. The mixtures were nutated for 12 hours at room temperature.

The volume of the mixtures was made up to 10 ml in each tube with LB3JD buffer. The magnetic beads were then immobilized on the magnetic stand. The supernatant (approximately 10ml) represents the unhybridised fraction and can be saved for separate analysis later. The pellet represents the pulled down hybridised chromatin target. The pellet was washed seven times with 10ml of LB3JD buffer. After each wash the beads were gently resuspended by nutation. Following the wash steps the beads were resuspended in 2.4ml of LB3JD buffer per tube and transferred to 2 Eppendorf (1.5ml) low binding tubes/pull-down.

The magnetic beads were immobilized using an Eppendorf magnetic stand. The supernatant was discarded and the beads were resuspended in 1ml of LB3JD buffer. The tubes were incubated for 5 minutes at 42°C in a thermomixer (shaking at 1000rpm) and then the beads were again immobilised using the Eppendorf magnetic stand. This step was repeated before the beads were finally resuspended in 0.6 ml of LB3JD buffer. The beads from each hybridization (telomere and control, 2 tubes each) were pooled in one tube/reaction and immobilized on the Eppendorf magnetic stand. The pellet was resuspended into 1 ml of Elution buffer (12.5mM Biotin (Invitrogen); 7.5 mM HEPES-NaOH pH 7.9; 75 mM NaCl; 1.5 mM EDTA pH 8; 0.75 mM EGTA pH 8; 0.15 % SDS; 0.075 % Sarkosyl) and incubated for 60 minutes at room temperature in the thermomixer (shaking at 1000rpm). Following incubation for 60 minutes the temperature was elevated to 65C and the mixtures were incubated for 10 minutes in order to effect elution of the pulled down chromatin target sequences.

The tubes were again placed on the Eppendorf magnetic stand so as to immobilize the magenetic beads and the eluate retained and transferred to fresh 1.5ml Eppendorf tubes. The OD260 of the eluates was checked (should be 15-25 ng/µl) to verify that the probe had been eluted from the magnetic beads.

To each of the eluates 230 µl of 100% TCA was added, and the tubes were incubated on ice for 10 minutes. The pulled down chromatin solution was then spun down at 16000g for 15 minutes at room temperature, and ∼1.1 ml carefully removed by pipetting. The tubes were brought up to 1.5ml with -20°C cold acetone, agitated for 10 seconds and then spun down again at 16000g for 10 minutes at room temperature. The supernatant was discarded and a small white pellet was visible. The pellet was washed with cold acetone one further time and spun down at 16000g at room temperature for 10 minutes. The supernatant was discarded and the pellet allowed to air dry.

The pellet containing the pulled down target chromatin was resuspended in 50µl of crosslinking reversal solution (250mM Tris pH 8.8; 2% SDS; 0.5M 2-mercaptoethanol). The solution was then incubated at 99°C for 25 minutes and either loaded onto an SDS-PAGE gel or stored at -80°C pending further analysis (for example by mass spectrometry).

### Sucrose Buffer

0.3 M Sucrose; 10 mM HEPES-NaOH pH 7.9; 1% Triton X-100; 3 mM CaCl₂; 2 mM MgOAc (Magnesium Acetate)

Glycerol Buffer25% Glycerol; 10 mM HEPES-NaOH pH 7.9; 0.1 mM EDTA; 0.1 mM EGTA; 5 mM MgOAc (Magnesium Acetate)

### 4. Oligonucleotide probe design

The probes are constituted of a mixture of LNA and DNA residues and were 25 nucleotides long. In addition, they were labelled with the biotin analogue, desthiobiotin at their 5' terminus, with the label being linked to the probe sequence via an extra-long spacer group, typically of between 20 and 95 carbon atoms in length. Desthiobiotin was selected as the affinity label so that more gentle elution conditions could be used compared to biotin. The extra-long spacer is of considerable importance, as the chromatin immobilization strategy could be impaired by steric hindrance problems (see for instance: Sandaltzopoulos R, Blank T, Becker PB. EMBO J. 1994). Suitable extra-long spacer technologies are described in Morocho et al. (Nucleos. Nucleot. Nuc. Acids. (2003) 22(5-8):1439-41; and Methods Mol Biol. (2005);288:225-40).

In the example described above the telomere targeting probe sequence was:
5' TtAgGgTtAgGgTtAgGgTtAgGgt 3' [SEQ ID NO: 1]

The randomised/scrambled control probe sequence was:
5' GaTgTgGaTgTggAtGtGgAtgTgg 3' [SEQ ID NO: 2]

Where CAPITALIZED letters represent LNA residues and lower case letters were DNA residues. The desthiobiotinylated LNA containing oligonucleotide probes were synthesised to order by Fidelity Systems (Gaithersburg, MD, USA). The molecular spacer utilised in the above probes was 108 carbon atoms in length.

### 5. Purification of pulled down proteins

The pulled-down proteins were loaded on a Bis-Tris 12% acrylamide minigel (Invitrogen) and run at 100V until the loading dye exited the gel. The gel was then fixed stained with Colloidal Blue (Invitrogen) following manufacturer's instructions. 15-25 bands were cut all along the lane (covering the whole lane). These samples were submitted to the Taplin Harvard Mass Spectrometry Facility (THMS) for analysis and protein identification (see http://gygi.med.harvard.edu/facility/#Intro; Taplin Biological Mass Spectrometry Facility, Harvard Medical School, C-Building Room 517, 240 Longwood Ave., Boston, MA 02115, USA).

Typically this analysis involves the following steps:
a. In-gel digestion of gel bands/spots
b. Micro-capillary LC/MS/MS anaylsis
c. Protein database searching

### 6. Data analysis and web-based reporting of data

The results are provided by THMS in the form of a private access web-site database.

The condensed data link showed peptides identified from the sample along with the corresponding proteins they matched to. Peptide sequences that were found in other proteins listed in the database are allocated a number (i.e. +3) in a column labelled "red" (redundancies). The information in the condensed data link was created by sorting and filtering the data obtained from the Sequest database search or searches.

The proteins were listed for each sample with the requirement of two or more strong scoring peptides matched to it by the database-searching program Sequest supplemented by manual inspection of the data. Proteins listed that had only one matching peptide were listed for information; proteins that only had one matching peptide may be correct but are typically verified by further confirmation (by western-blot for instance)

The novel telomere chromatin associated factors identified are set out in Table 1.

### Example 2

The starting cells were the transformed human embryonic lung fibroblast cell line WI38 VA13 (Castellani et al. (1986) J. Cell. Biol. 103:1671-1677). Unlike the HeLa cell line used in Example 1, WI38 VA13 is a non-telomerase, ALT cell type. The protein and polypeptide factors associated with ALT telomere sequences were isolated according to the method described in Example 1.

The novel ALT telomere chromatin associated factors identified are set out in Table 2. Figure 2 shows a graphical comparison of the novel chromatin associated factors identified according to Examples 1 and 2. It shows that an area of overlap is evident in which certain factors are commonly associated with telomeric sequences in both telomerase and ALT cell types. The identities of the chromatin associated factors common to both telomerase cells (HeLa) and ALT cells (WI38 VA13) are shown in Table 3.

### Example 3

### Characterisation of novel chromatin associated factors identified by the method of the invention.

Experiments were performed to demonstrate that candidates identified by the method of the invention are functionally significant. The ALT cell line WI38 VA13 was transfected with two shRNA constructs (SIGMA, Mission shRNA collection) targeting the COUP-TF2 gene. COUP-TF2 is an orphan nuclear receptor identified as a novel chromatin associated factor in Example 2 (shown in bold in Table 2). Cells were co-transfected with a green fluorescent protein construct to identify those which had been successfully transfected. 96 hours post-transfection, the cells were fixed and dual stained with an anti-rap1 antibody (rabbit polyclonal ab4181, AbCam) to detect telomeres and an anti-PML antibody (mouse monoclonal PGM3, Santa Cruz) to identify PML-bodies. The localisation signal was developed using donkey anti-rabbit-Cy3 and donkey anti-mouse-Cy5 polyclonal antibodies (Jackson Labs).

As shown in Figure 3, PML and Rap1 signals co-localise in untransfected cells (identified by lack of GFP staining). However, in GFP-positive cells, the two signals no longer co-localise. This demonstrates that COUP-TF2 is required for localizing telomeres to PML bodies in ALT cells. PML bodies have been associated with many nuclear functions including: transcription, DNA repair, viral defence, stress, cell cycle regulation, proteolysis and apoptosis. Hence, the ability to inhibit localisation of telomeres to PML bodies in cancer cells may provide an important route to cancer therapy.

Further knockdown experiments using shRNAs specific for the TR4 gene (another novel factor identified herein) have shown the similar results as for COUP-TF2. These results suggest that COUP-TF2 and/or TR4 can act as critical mediators of telomere localisation to PML bodies in vivo.

Further experiments (not shown) have demonstrated that these effects are not cell line-specific as knockdowns of COUP-TF2 or TR4 have exactly the same effect in the Saos-2 osteosarcoma ALT cell line, as in the WI38VA13 cells.

Using the methodology of the invention for targeting of polypeptides and proteins that associate with telomeric sequences resulted in the identification of a significant number of associated proteins.. Nevertheless, the methods of the invention also demonstrate utility when adapted to target single copy sequences, where it is anticipated that smaller numbers of proteins may be identified. In these cases, the methods of the invention lead to identification of interaction pathways that can be used to develop novel hypotheses within a systems biology framework, and hence identify the most chemically tractable components of a gene control pathway.

### Example 4

### Preparation of the chromatin template by ultra-sonication

The present example provides an alternative method for preparation of the chromatin fragments to that described in Example 1.

The following volumes and numbers are given for one purification (that is ∼3.10⁹ cell equivalent): Cells were centrifuged at 2000g for 2 minutes at room temperature and pellet was resuspended into the same pellet volume of 1X PBS-0.5%Triton X-100 and 90 µl of RNaseA (Qiagen 100mg/ml) were added. Cells were incubated for 60 minutes at room temperature with shaking then at 4°C for 12-16 h. Cells were washed 6 times in PBS. Cells were equilibrated in LBJD solution (10 mM HEPES-NaOH pH 7.9; 100 mM NaCl; 2mM EDTA pH 8; 1 mM EGTA pH 8; 0.2% SDS; 0.1% Sarkosyl, protease inhibitors) and pellet was resuspended into 55% pellet volume of LBJD solution. Samples were sonicated (Micro-tip, Misonix 3000) using the following parameters: Power setting 7 (36-45 Watts), 15 seconds constant pulse and 45 seconds pause for a 7 minutes total process time. Sample was collected by centrifugation at 16000g for 15 minutes at room temperature. Chromatin sample was then applied to Sephacryl S-400-HR spin columns and incubated at 58°C for 5 minutes.

LBJDLS (10 mM HEPES-NaOH pH 7.9; 30 mM NaCl; 2 mM EDTA pH 8; 1 mM EGTA pH 8; 0.2% SDS; 0.1% Sarkosyl, protease inhibitors) pre-equilibrated streptavidin beads were added (Pierce Ultralink streptavidin, 0.5ml) and the sample was incubated for 2h at room temperature. Beads were discarded and supernatant was saved.

**Table 1 - Telomere associated chromatin binding factors from a HeLa cell line**

| **Short form name/Uniprot entry name** | **Description of factor** | **Previously known to associate with telomere chromatin?** |
|---|---|---|
| U5S1 | 116 kda u5 small nuclear ribonucleoprotein component | N |
| Q9P037 | 14.3.3 eta | N |
| 1433G | 14.3.3 Gamma | N |
| PSD10 | 26s proteasome non-atpase regulatory subunit 10 | N |
| PSD5 | 26s proteasome non-atpase regulatory subunit 5 | N |
| AN32B | acidic leucine-rich nuclear phosphoprotein 32 family member b | N |
| AN32E | acidic leucine-rich nuclear phosphoprotein 32 family member e | N |
| AY892677_1 | ADP-ribosylation factor 3 | N |
| ARF5 | adp-ribosylation factor 5 | N |
| APEX1 | ap endonuclease 1 (Component of the SET complex, which also contains SET, ANP32A, HMGB2 and NME1) | N |
| AURKB | Aurora-B | N |
| Q15394 | Basic leucine zipper and W2 domain-containing protein 1 | N |
| BOREA | borealin | N |
| CYBP | Calcyclin-binding protein (s100 binding protein) | N |
| CLIC1 | chloride channel nuclear | N |
| RCC1 | chromosome condensation protein 1 | N |
| STAG2 | cohesin subunit sa-2 (stromal antigen 2) (scc3 homolog 2) | N |
| DBPA | Cold shock domain-containing protein A | N |
| CIRBP | Cold-inducible RNA-binding protein (UV repair protein ?) | N |
| CND3 | condensin complex subunit 3 (chromosome-associated protein g) | N |
| Q5HYH5 | cpsf5 | N |
| CPSF6 | cpsf6 (in paraspeckles) | N |
| Q5U5J2 | CSNK2A1 protein (kinase) | N |
| CAND1 | Cullin-associated NEDD8-dissociated protein 1 | N |
| Q4R5B9_MACFA | Cyclin A/CDK2-associated protein p19 | N |
| DDX1 | ddx1 | N |
| OTUB1 | Deubiquitinating enzyme OTUB1 | N |
| RPA2 | dna-directed rna polymerase i 135 kda polypeptide | N |
| RPA1 | dna-directed rna polymerase i largest subunit | N |
| RPF53 | dna-directed rna polymerase i-associated factor 53 kda subunit | N |
| RPB5 | dna-directed rna polymerase ii 23 kda polypeptide | N |
| XRCC1 | dna-repair protein xrcc1 (x-ray repair cross-complementing protein 1 | N |
| DCTN2 | dynactin subunit 2 | N |
| ELAV1 | ELAV-like protein 1 | N |
| PA2G4 | ErbB3-binding protein 1 (binds AR and NCL) | N |
| Q7Z4L1 | eukaryotic initiation factor 5a isoform i variant a | N |
| Q53HJ5 | eukaryotic translation initiation factor 5a variant | N |
| IF6 | eukaryotic translation initiation factor 6 ( | N |
| XPO1 | exportin-1 (exp1) (chromosome region maintenance 1 protein homolog). | N |
| FA50A | FAM50A | N |
| FUBP1 | Far upstream element-binding protein 1 | N |
| FUBP2 | far upstream element-binding protein 2 | N |
| FBRL | fibrillarin(rRNA 2'-O-methyltransferase fibrillarin) | N |
| LEG7 | Galectin-7 | N |
| GRWD1 | Glutamate-rich WD repeat-containing protein 1 | N |
| AB002283_1 | hMBF1b | N |
| HMG1X | hmg1l10 | N |
| HDGF | hmg1l2 | N |
| Q5T6U9 | HMGA1 | N |
| Q75MM1 | hmgb1 | N |
| Q96J53 | hmgb2 protein | N |
| HMGB3 | hmgb3 protein | N |
| Q5SWC6 | hp1-bp74 (fragment) | N |
| Q59FT0 | hypothetical protein | N |
| Q9UFC0 | Hypothetical protein DKFZp434K1815 | N |
| Q53RR5 | hypothetical protein ywhaq | N |
| IMB1 | importin beta-1 subunit | N |
| 075141 | kiaa0650 protein (SMCHD1) | N |
| LRC41 | Leucine-rich repeat-containing protein 41 | N |
| Q5VLR4 | Lung cancer oncogene 7 | N |
| PIR | makes dots in the nucleus | N |
| WDR61 | Meiotic recombination REC14 protein homolog (check out ski8 exosomes etc..) | N |
| Q8NI90 | member ras oncogene family. | N |
| NACA | Nascent polypeptide-associated complex subunit alpha interacts with smyd1 | N |
| SEPT_2 | NEDD5 | N |
| NUDC | Nuclear distribution protein C homolog | N |
| NUMA1 | nuclear mitotic apparatus protein 1 (numa protein) | N |
| UBF1 | Nucleolar transcription factor 1 | N |
| ORC1 | origin recognition complex | N |
| ORC2 | origin recognition complex | N |
| ORC3 | origin recognition complex | N |
| ORC4 | origin recognition complex | N |
| ORC5 | origin recognition complex | N |
| PARK7 | Parkinson disease protein 7 binds AR, DAXX | N |
| PIN1 | peptidyl-prolyl cis-trans isomerase nima-interacting 1 | N |
| PCNP | pest-containing nuclear protein | N |
| PP1G | PP1c gamma | N |
| SMCA1 | probable global transcription activator snf2I1 | N |
| Q99834 | Programmed cell death protein 4 (Nuclear antigen H731-like) | N |
| PDCD6 | Programmed cell death protein 6 | N |
| PSA7 | Proteasome subunit alpha type 7 | N |
| PSA7L | proteasome subunit alpha type 7-like | N |
| PSB6 | proteasome subunit beta type 6 precursor | N |
| PIAS1 | protein inhibitor of activated stat protein 1 | N |
| RCC2 | protein rcc2 (telophase disk protein of 60 kda) | N |
| PTD4 | Putative GTP-binding protein 9 | N |
| PEBP1 | Raf kinase inhibitor protein | N |
| AB051846_1 | Raichu404X-ras rab related | N |
| RAB10 | ras-related protein rab-10 | N |
| RAB35 | ras-related protein rab-35 | N |
| RAB5C | ras-related protein rab-5c | N |
| RAB6A | ras-related protein rab-6a ( | N |
| Q5T3J3 | receptor-interacting factor 1 protein | N |
| Q6PAU7 | ribosomal prot related | N |
| FUS | rna-binding protein fus (oncogene fus) promotes D-loop formation | N |
| FKBP5 | rotamase | N |
| PPIB | rotamase (prolyl-isomerase) | N |
| PRP4B | Serine/threonine-protein kinase PRP4 homolog (interacts with snw1) | N |
| PP1RA | Serine/threonine-protein phosphatase 1 regulatory subunit 10 makes dots in the nucleus | N |
| SNW1 | SNW domain-containing protein 1 (Involved in vitamin D-mediated transcription. Can function as a splicing factor in pre-mRNA splicing.) | N |
| SF3B1 | Splicing factor 3B subunit 1 | N |
| SF3B3 | splicing factor 3b subunit 3 | N |
| SFRS9 | splicing factor, arginine/serine-rich 9 | N |
| SND1 | Staphylococcal nuclease domain-containing protein 1 (contains tudor domains, RISC member in elegans) | N |
| SMC1A | structural maintenance of chromosome 1-like 1 protein | N |
| SMC3 | structural maintenance of chromosome 3 ( | N |
| TADBP | TAR DNA-binding protein 43 | N |
| RBP56 | tata-binding protein-associated factor 2n | N |
| Q96QB7 | tc4 protein (ran) | N |
| THOC4 | tho complex subunit 4 | N |
| TF3C3 | Transcription factor IIIC-subunit gamma | N |
| TFR1 | transferrin receptor protein 1 | N |
| TAGL2 | Transgelin-2 (nuclear membrane) | N |
| TMEDA | transmembrane emp24 domain-containing protein 10 precursor | N |
| TCOF | Treacher Collins syndrome protein | N |
| Q9NXD9 | tRNA (cytosine-5-)-methyltransferase NSUN2 | N |
| RU2A | U2 small nuclear ribonucleoprotein A' (interacts with hnf1a) | N |
| UBE1 | Ubiquitin-activating enzyme E1 | N |
| KCY | UMP-CMP kinase | N |
| CN021 | Uncharacterized protein C14orf21 (contains pumillo repeats) | N |
| Q5VU13 | V-set and immunoglobulin domain-containing protein 10 | N |
| Q6UXN9 | WD repeat protein 82 (interacts with CUL4B) | N |
| Q2F831 | YWHAZ. | N |
| WRIP1 | atpase wrnip1 (werner helicase-interacting protein) | Y |
| DHX15 | dhx15, interacts with LA | Y |
| NAT10 | n-acetyltransferase 10 | Y |
| RAD50 | dna repair protein rad50 | Y |
| NBN | NBS1 | Y |
| PARP1 | parp1 protein | Y |
| NOLA1 | snoRNP at telomere | Y |
| NOLA2 | snoRNP at telomere | Y |
| SP100 | sp100 protein | Y in ALT cells |

**Table 2 - Telomere associated chromatin binding factors from the WI38 VA13 cell line (an ALT phenotype cell line)**

| **Short form name/Uniprot entry name** | **Description of factor** | **Previously known to associate with telomere chromatin?** |
|---|---|---|
| Q6LD62 | 14-3-3 protein/cytosolic phospholipase a2 protein | N |
| AN32E | acidic leucine-rich nuclear phosphoprotein 32 family member e | N |
| API5 | apoptosis inhibitor 5 ( | N |
| ATRIP | ATR interacting protein | N |
| BTBDC | btb/poz domain-containing protein 12 | N |
| Q96E14 | c16orf75 protein | N |
| Q5VYV7 | C20orf94 protein | N |
| Q6NXN8 | C5orf25 protein | N |
| Q96HY3 | calm 1 protein | N |
| Q9BRL5 | calm3 | N |
| PEBB | CBF-beta | N |
| Q9UFW8 | CGG triplet repeat binding protein 1 | N |
| CHM4B | charged multivesicular body protein 4b (chromatin-modifying protein 4b | N |
| CAF1B | chromatin assembly factor 1 subunit b | N |
| C1QBP | complement component 1 q subcomponent-binding protein | N |
| CCNA2 | Cyclin A2 | N |
| DLDH | dihydrolipoyl dehydrogenase, mitochondrial precursor | N |
| PRI2 | dna primase large subunit | N |
| DNJA1 | dnaj homolog subfamily a member 1 | N |
| DNJA2 | dnaj homolog subfamily a member 2 | N |
| DNJC9 | dnaj homolog subfamily c member 9 ( | N |
| EAR2 | ear2, nr2f6 | N |
| EMD | emerin | N |
| ETHE1 | ethe1 protein | N |
| Q53SC4 | Ewing's tumor-associated antigen 1 | N |
| FANCJ | Fanconi Anemia Member J | N |
| Q9BQA8 | g patch domain and kow motifs | N |
| LEG1 | galectin-1 | N |
| TRAP1 | heat shock protein 75 kda | N |
| HMG1X | high mobility group protein 1-like 10 (hmg-1110 | N |
| HMGB3 | high mobility group protein b3 | N |
| HINT2 | Hint2 | N |
| HDAC2 | histone deacetylase 2 (hd2) | N |
| Q75MM1 | hmg1 | N |
| ITAX | integrin alpha-x precursor | N |
| UCRP | interferon-induced 17 kda protein precursor | N |
| IDH3A | isocitrate dehydrogenase | N |
| SAM68 | kh domain-containing, rna-binding, signal transduction-associated protein 1 ( | N |
| ADPPT | I-aminoadipate-semialdehyde dehydrogenase-phosphopantetheinyl transferase | N |
| Q5T974 | LEM domain-containing protein 2 | N |
| LASP1 | lim and sh3 domain protein 1 (lasp-1) | N |
| LONM | Ion protease homolog | N |
| PPAC | low molecular weight phosphotyrosine protein phosphatase | N |
| LSD1 | lysine-specific histone demethylase 1 | N |
| AB002283_1 | MBF1beta | N |
| MTA1 | metastasis-associated protein mta1 | N |
| Q9UJ54 | Methionine adenosyltransferase II beta | N |
| MBD3 | methyl-cpg-binding domain protein 3 | N |
| MORC3 | morc3, nxp-2 | N |
| NELFB | negative elongation factor b (nelf-b) (cofactor of brca1) | N |
| NAMPT | nicotinamide phosphoribosyltransferase | N |
| | | |
| NRIP1 | nuclear receptor-interacting protein 1 | N |
| NUCL | nucleolin | N |
| TR2 | orphan nuclear receptor tr2 | N |
| PTMS | parathymosin. | N |
| PEBP1 | phosphatidylethanolamine-binding protein 1 | N |
| PIAS1 | PIAS1 | N |
| PIAS3 | PIAS3 | N |
| PP25 | placental protein p25 | N |
| PNKP | polynucleotide phosphatase/kinase (polynucleotide kinase- 3'-phosphatase) interacts with XRCC1 | N |
| Q53FV0 | prohibitin variant | N |
| PIN1 | Prolyl isomerase Pin1 | N |
| FA50A | protein fam50a | N |
| NIPS1 | protein nipsnap1. | N |
| PPME1 | protein phosphatase methylesterase 1 | N |
| AF054183_1 | ran | N |
| Q5T3J3 | receptor-interacting factor 1 | N |
| RCC1 | regulator of chromosome condensation (chromosome condensation protein 1) | N |
| AB209732_1 | replication protein A1, 70kDa variant | N |
| RCOR1 | rest corepressor 1 (protein corest) | N |
| GLYC | serine hydroxymethyltransferase | N |
| GLYM | serine hydroxymethyltransferase, mitochondrial precursor | N |
| SPB12 | serpin b12. | N |
| SIAS | sialic acid synthase | N |
| Q7Z5A3 | small nuclear ribonucleoprotein sm d1 | N |
| SMD3 | small nuclear ribonucleoprotein sm d3 ( | N |
| 075141 | smchd1 | N |
| STMN2 | stathmin-2 | N |
| ERR1 | steroid hormone receptor err1 | N |
| STML2 | stomatin-like protein 2 (slp-2) | N |
| STIP1 | stress-induced-phosphoprotein 1 | N |
| SODM | superoxide dismutase | N |
| TADBP | TAR DNA binding protein 23 | N |
| TCPH | t-complex protein 1 subunit eta | N |
| KITH | thymidine kinase, cytosolic | N |
| P66B | transcriptional repressor p66 beta (p66/p68) | N |
| TAGL2 | transgelin-2 | N |
| PRP31 | u4/u6 small nuclear ribonucleoprotein prp31 | N |
| U33K | uba/ubx 33.3 kda protein | N |
| UCHL1 | ubiquitin carboxyl-terminal hydrolase isozyme I1 | N |
| Q7KZS0 | ubiquitin-conjugating enzyme e2i (ubc9 homolog, yeast) | N |
| UAP1 | udp-n-acetylhexosamine pyrophosphorylase | N |
| CT077 | uncharacterized protein c20orf77 | N |
| Q17R98 | unknown | N |
| IF4H | williams-beuren syndrome chromosome region 1 protein | N |
| Q53RR5 | ywhaq | N |
| Q8N279 | Zinc Finger protein 64 | N |
| ZA2G | zinc-alpha-2-glycoprotein precursor | N |
| RMI1 | protein rmi1 homolog | N |
| SSB | single-stranded dna-binding protein, mitochondrial precursor (mt-ssb) | N |
| RECQ1 | atp-dependent dna helicase q1 | N |
| COT1 | coup-tf1 | N |
| **COT2** | **coup-tf2** | **N** |
| **TR4** | **orphan nuclear receptor tr4** | **N** |
| ERCC1 | dna excision repair protein ercc-1 | Y |
| ERCC4 | dna repair endonuclease xpf | Y |
| RAD50 | dna repair protein rad50 | Y |
| PARP1 | poly [adp-ribose] polymerase 1 | Y |
| RFC2 | replication factor c | Y |
| RFC4 | replication factor c | Y |
| BLM | Bloom helicase | Y in ALT |
| RAD9A | cell cycle checkpoint control protein rad9a | Y in ALT |
| HUS1 | checkpoint protein hus1 | Y in ALT |
| RAD1 | DNA repair exonuclease rad1 homolog | Y in ALT |
| Q96MF7 | MMS21 homolog | Y in ALT |
| Q96SB8 | smc6 protein | Y in ALT |
| TOP3A | topoisomerase 3 alpha | Y in ALT |
| SP100 | nuclear autoantigen sp-100 | Y in ALT |
| PML | pml | Y in ALT |
| H2A1D | histone h2a type 1-d | Y |
| Q6FGB8 | histone h4 | Y |
| Q99525 | histone h4 | Y |
| Q6DRA9 | histone h4/o | Y |

**Table 3 Telomere associated chromatin binding factors identified as common to both the HeLa (telomerase phenotype) and WI38 VA13 (ALT phenotype) cell line**

| **Short form name/Uniprot entry name** | **Description of factor** | **Previously known to associate with telomere chromatin?** |
|---|---|---|
| 1433B | 14.3.3 Beta | N |
| BC051814_1 | 14.3.3 zeta | N |
| Q4VJB6 | 14-3-3 protein epsilon isoform transcript variant 1 | N |
| ARF4 | adp-ribosylation factor 4 protein | N |
| HCC1 | cip29 | N |
| CLIC1 | nuclear chloride channel | N |
| Q71V99 | cyclophilin | N |
| CSRP1 | Cysteine and glycine-rich protein 1 | N |
| DAXX | daxx | N |
| DUT | Deoxyuridine 5'-triphosphate nucleotidohydrolase | N |
| DDB1 | dna damage-binding protein 1 (damage-specific dna-binding protein 1) | N |
| DNJB1 | dnaj homolog subfamily b member 1 | N |
| DNJC8 | dnaj homolog subfamily c member 8 | N |
| Q96FD2 | enos interacting protein | N |
| IF5A2 | eukaryotic translation initiation factor 5a-2 | N |
| XPO2 | exportin-2 (exp2) (importin-alpha re-exporter) (chromosome segregation 1-like protein | N |
| SPT16 | fact | N |
| SSRP1 | fact | N |
| GUAA | gmp synthase | N |
| HAT1 | histone acetyltransferase type b catalytic subunit | N |
| RBBP7 | histone-binding protein rbbp7 (retinoblastoma-binding protein 7) | N |
| Q5D862 | ifapsoriasin (filaggrin 2 | N |
| H2AY | macroH2A | N |
| BUB3 | Mitotic checkpoint protein BUB3 (WD repeats) | N |
| Q567Q0 | peptidylprolyl isomerase a, isoform 2 | N |
| PRP19 | pre-mrna-splicing factor 19 (prp19/pso4 homolog) (nuclear matrix protein 200) | N |
| ANM1 | PRMT-1 protein | N |
| PHB2 | prohibitin-2 | N |
| PSME3 | proteasome activator complex subunit 3 | N |
| CN166 | Protein C14orf166 | N |
| SET | Protein SET (interacts with ANP32A, APEX1, HMGB2 and NME1, but not NME2). | N |
| RAB1B | ras-related protein rab-1 b | N |
| GDIR | rho-gdi alpha | N |
| DDX5 | rna helicase p68 | N |
| RBM8A | rna-binding protein 8a | N |
| SRP14 | signal recognition particle 14 kda protein | N |
| UAP56 | spliceosome rna helicase bat1 ( | N |
| SUMO3 | sumo-3 | N |
| SMCA5 | swi/snf-related matrix-associated actin-dependent regulator of chromatin subfamily a member 5 | N |
| TCPB | t-complex protein 1 subunit beta | N |
| TCPD | t-complex protein 1 subunit delta | N |
| TCPE | t-complex protein 1 subunit epsilon | N |
| TCEA1 | Transcription elongation factor S-II protein 1 | N |
| TIF1B | transcription intermediary factor 1-beta | N |
| TERA | Transitional endoplasmic reticulum ATPase (interacts with WRN helicase) | N |
| TM109 | transmembrane protein 109 precursor | N |
| UBP7 | ubiquitin carboxyl-terminal hydrolase 7 | N |
| ULE1A | ubiquitin-like 1-activating enzyme e1a (sumo-1-activating enzyme subunit 1) | N |
| Q8WV43 | unknown contains wd40 repeats (vague homology to CSA, xrcc8) | N |
| IF4H | williams-beuren syndrome chromosome region 1 protein | N |
| CDC73 | parafibromin (cell division cycle protein 73 homolog) | N |
| OBFC1 | ob fold containing protein 1 | N |
| HMBX1 | hmbox1 protein | N |
| CBX5 | hp1 alpha | N in human |
| CBX1 | hp1 beta | N in human |
| CBX3 | hp1 gamma | N in human |
| LA | autoantigen LA | Y |
| DCR1B | Appolo | Y |
| MSH2 | dna mismatch repair protein msh2 (muts protein homolog 2) | Y |
| PRKDC | DNA-PKc | Y |
| MRE11 | double-strand break repair protein mre11a | Y |
| FEN1 | flap endonuclease 1 | Y |
| KU70 | ku proteins | Y |
| KU86 | ku proteins | Y |
| MCM2 | mem protein | Y |
| MCM3 | mem protein | Y |
| MCM4 | mem protein | Y |
| MCM5 | mem protein | Y |
| MCM6 | mem protein | Y |
| MCM7 | mem protein | Y |
| PCNA | pcna | Y |
| POTE1 | POT1 protein | Y |
| TE2IP | rap1 protein | Y |
| RFA1 | replication factor a | Y |
| RFA2 | replication factor a | Y |
| RFC5 | replication factor c subunit 5 | Y |
| TINF2 | TIN2 protein | Y |
| ACD | TPP1 protein | Y |
| TERF1 | trf1 protein | Y |
| TERF2 | trf2 protein | Y |
| CDC2 | cdk1 protein | Y in yeast |
| H2A1A | histone h2a type 1-a. | Y |
| H2A1B | histone h2a type 1-b | Y |
| H2B1A | histone h2b type 1-a (histone h2b, testis) (testis-specific histone h2b) | Y |
| H2B1B | histone h2b type 1-b (h2b.f) | Y |
| H2B1C | histone h2b type 1-c/e/f/g/i | Y |
| H3T | histone h3 | Y |

## Claims

1. A method for isolating one or more polypeptides associated with a target nucleic acid sequence comprising:
(a) obtaining a sample that comprises a target nucleic acid sequence and one or more polypeptides associated with the target nucleic acid sequence;
(b) contacting the sample with at least one oligonucleotide probe that comprises a sequence that is complimentary to and capable of hybridising with at least a portion of the target nucleic acid sequence, wherein the oligonucleotide probe comprises at least one locked nucleic acid (LNA) nucleotide and wherein the oligonucleotide probe further comprises at least one affinity label, wherein the affinity label is conjugated to the oligonucleotide probe via a spacer group having a length of between 40 and 170 atoms; more suitably between 70 and 130 atoms; more typically between 90 and 120 atoms; suitably between 100 and 110 atoms;
(c) allowing the at least one oligonucleotide probe and the target nucleic acid sequence to hybridise with each other so as to form a probe-target hybrid;
(d) isolating the probe-target hybrid from the sample by immobilizing the probe-target hybrid through a molecule that binds to the at least one affinity label; and
(e) eluting the one or more polypeptides that are associated with the target nucleic acid sequence.

2. The method of claim 1, wherein the target nucleic acid sequence comprises eukaryotic DNA, such as mammalian DNA, or prokaryotic DNA, or RNA, or viral derived sequences integrated into mammalian DNA.

3. The method of claim 2, wherein the target nucleic acid sequence is comprised within chromatin.

4. The method of claim 3, wherein the one of more polypeptides that are associated with the target nucleic acid sequence are chromatin associated polypeptides.

5. The method of any previous claim, wherein the affinity label is selected from the group consisting of biotin or an analogue thereof, including desthiobiotin; digoxigenin; fluorescein; dinitrophenol; and an immunotag.

6. The method of any previous claim, wherein the probe-target hybrid is immobilized through a molecule that binds to the at least one affinity label and which molecule is attached to a solid substrate.

7. The method of claim 6, wherein the solid substrate comprises a microbead, and wherein the microbead is capable of being magnetically separated from a solution.

8. The method of any previous claim, wherein the oligonucleotide probe includes 50% LNA nucleotide residues.

9. The method of any previous claim, wherein the one or more polypeptides associated with the target nucleic acid sequence are exposed to conditions that result in crosslinking of the one or more polypeptides prior to the step of exposing the sample to the oligonucleotide probe, and wherein the crosslinking is reversed prior to the step of eluting the one or more polypeptides.

10. The method of any previous claim further comprising an additional step of:
(f) analysing the eluted one or more polypeptides via a procedure that includes mass spectrometry to determine their identity.

11. An oligonucleotide probe comprising at least one group that conforms to general formula I, set out below:
A - [C]ₙ - X I
wherein A includes one or more affinity labels tethered to a nucleotide X by a spacer group of n atoms in length, wherein n is between 90 and 170 atoms; A comprises a hapten or an immuno-tag; and wherein the nucleotide X is a locked ribonucleotide (LNA).

12. An oligonucleotide probe that conforms to general formula II, set out below:
B - [C]ₙ - Y II
wherein B is an affinity label that is tethered to oligonucleotide sequence Y via a spacer group comprising a linear chain of n atoms, wherein n is between 90 and 170 atoms; the oligonucleotide sequence Y comprising at least 10 nucleotides of which at least 10% and up to 50% are locked nucleic acid (LNA) nucleotides.

13. An oligonucleotide probe that comprises a group that conforms to general formula III, set out below: 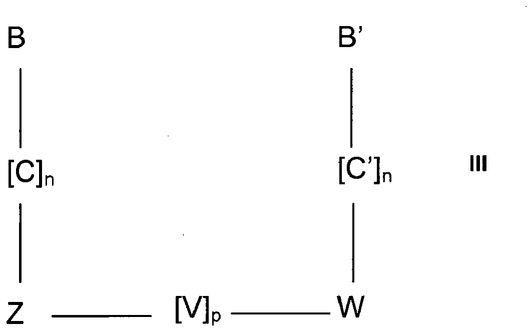 wherein B and B' include an affinity label that is the same or different tethered to respective nucleotides Z and W by spacer groups C and C' of n atoms in length wherein n is between 90 and 170 atoms; and wherein the nucleotides Z and W are separated by an oligonucleotide chain V of p nucleotides in length, where p is between 0 and 40; the nucleotides Z, W and V being the same or different and selected suitably from a ribonucleotide, a deoxyribonucelotide, a dideoxyribonucleotide and a locked ribonucleotide (LNA), and at least one of Z and W is a LNA.

14. The oligonucleotide probes of claims 11 to 13, wherein the affinity label is selected from one of the group consisting of biotin or an analogue thereof, including desthiobiotin; digoxigenin; fluorescein; and dinitrophenol.

## Patentansprüche

1. Verfahren zum Isolieren eines oder mehrerer Polypeptide, die mit einer Zielnukleinsäuresequenz verbunden sind, Folgendes umfassend:
(a) Erhalten einer Probe, die eine Zielnukleinsäuresequenz und ein oder mehrere Polypeptide umfasst, die mit der Zielnukleinsäuresequenz verbunden sind;
(b) In-Berührung-Bringen der Probe mit wenigstens einer Oligonukleotidsonde, die eine Sequenz umfasst, welche komplementär ist zu wenigstens einem Teil der Zielnukleinsäuresequenz und mit diesem hybridisieren kann, wobei die Oligonukleotidsonde wenigstens ein LNA-Nukleotid (locked nucleic acid) umfasst und wobei die Oligonukleotidsonde ferner wenigstens ein Affinitätskennzeichen trägt, wobei das Affinitätskennzeichen über eine Abstandhaltergruppe mit einer Länge zwischen 40 und 170 Atomen mit der Oligonukleotidsonde konjugiert ist, besser geeignet zwischen 70 und 130 Atomen, üblicherweise zwischen 90 und 120 Atomen, geeigneterweise zwischen 100 und 110 Atomen;
(c) Zulassen, dass die wenigstens eine Oligonukleotidsonde und die Zielnukleinsäuresequenz miteinander hybridisieren, um ein Sonde-Ziel-Hybrid auszubilden;
(d) Isolieren des Sonde-Ziel-Hybrids von der Probe durch Immobilisieren des Sonde-Ziel-Hybrids durch ein Molekül, das mit dem wenigstens einen Affinitätskennzeichen bindet; und
(e) Eluieren des einen oder der mehreren Polypeptide, die mit der Zielnukleinsäuresequenz verbunden sind.

2. Verfahren nach Anspruch 1, wobei die Zielnukleinsäuresequenz eukaryotische DNA, etwa Säugetier-DNA, oder prokaryotische DNA oder RNA oder in Säugetier-DNA integrierte, von Viren abgeleitete Sequenzen umfasst.

3. Verfahren nach Anspruch 2, wobei die Zielnukleinsäuresequenz in Chromatin enthalten ist.

4. Verfahren nach Anspruch 3, wobei das eine oder die mehreren Polypeptide, die mit der Zielnukleinsäuresequenz verbunden sind, chromatinassoziierte Polypeptide sind.

5. Verfahren nach einem der vorhergehenden Ansprüche, wobei das Affinitätskennzeichen ausgewählt ist aus der Gruppe bestehend aus Biotin oder einem Analogon desselben, einschließlich Desthiobiotin, Digoxigenin, Fluorescein, Dinitrophenol und einem Immuno-Tag.

6. Verfahren nach einem der vorhergehenden Ansprüche, wobei das Sonde-Ziel-Hybrid durch ein Molekül immobilisiert wird, das an dem wenigstens einen Affinitätskennzeichen bindet, wobei das Molekül mit einem festen Substrat verbunden ist.

7. Verfahren nach Anspruch 6, wobei das feste Substrat ein Microbead umfasst, und wobei das Microbead magnetisch aus einer Lösung abgesondert werden kann.

8. Verfahren nach einem der vorhergehenden Ansprüche, wobei die Oligonukleotidsonde 50 % LNA-Nukleotidrückstände umfasst.

9. Verfahren nach einem der vorhergehenden Ansprüche, wobei das eine oder die mehreren Polypeptide, die mit der Zielnukleinsäuresequenz verbunden sind, Bedingungen ausgesetzt werden, die vor dem Schritt, in dem die Probe der Oligonukleotidsonde ausgesetzt wird, zum Vernetzen des einen oder der mehreren Polypeptide führen, und wobei das Vernetzen vor dem Schritt des Eluierens des einen oder der mehreren Polypeptide umgekehrt wird.

10. Verfahren nach einem der vorhergehenden Ansprüche, ferner umfassend den folgenden zusätzlichen Schritt:
(f) Analysieren des einen oder der mehreren eluierten Polypeptide durch einen Vorgang, der Massenspektrometrie einschließt, um ihre Identität zu bestimmen.

11. Oligonukleotidsonde, umfassend wenigstens eine Gruppe, die der folgenden allgemeinen Formel I entspricht:
**A - [C]ₙ - X** **I**
wobei A ein oder mehrere Affinitätskennzeichen umfasst, die über eine Abstandhaltergruppe mit einer Länge von n Atomen an ein Nukleotid X gebunden sind, wobei n zwischen 90 und 170 Atomen liegt; A ein Hapten oder einen Immuno-Tag aufweist, und wobei das Nukleotid X ein LNA-Ribonukleotid ist.

12. Oligonukleotidsonde, die der folgenden allgemeinen Formel II entspricht:
**B - [C]ₙ - Y** **II**
wobei B ein Affinitätskennzeichen ist, das über eine Abstandhaltergruppe, die eine lineare Kette von n Atomen umfasst, an die Oligonukleotidsequenz Y gebunden ist, wobei n zwischen 90 und 170 Atomen liegt; wobei die Oligonukleotidsequenz Y wenigstens 10 Nukleotide umfasst, von denen wenigstens 10 % und bis zu 50 % LNA-Nukleotide sind.

13. Oligonukleotidsonde, umfassend eine Gruppe, welche der folgenden allgemeinen Formel III entspricht: 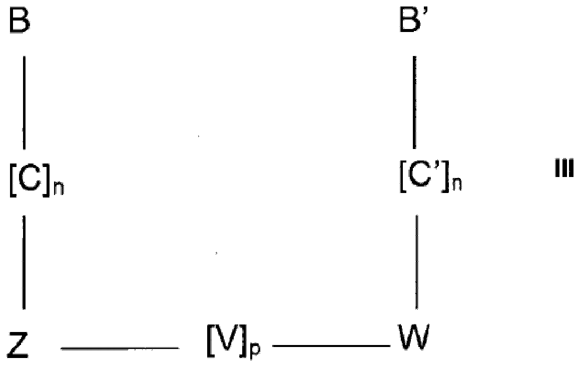 wobei B und B' ein Affinitätskennzeichen enthalten, welches gleich oder unterschiedlich ist und über die Abstandhaltergruppen C und C' mit einer Länge von n Atomen mit entsprechenden Nukleotiden Z und W verbunden ist, wobei n zwischen 90 und 170 Atomen liegt; und wobei die Nukleotide Z und W durch eine Oligonukleotidkette V mit einer Länge von p Nukleotiden voneinander getrennt sind, wobei p zwischen 0 und 40 liegt; wobei die Nukleotide Z, W und V gleich oder unterschiedlich sind und angemessen ausgewählt sind aus einem Ribonukleotid, einem Desoxyribonukelotid, einem Didesoxyribonukleotid und einem LNA-Ribonukleotid und wobei Z und/oder W ein LNA sind/ist.

14. Oligonukleotidsonden nach den Ansprüchen 11 bis 13, wobei das Affinitätskennzeichen ausgewählt ist aus der Gruppe bestehend aus Biotin oder einem Analogon desselben, einschließlich Desthiobiotin, Digoxigenin, Fluorescein und Dinitrophenol.

## Revendications

1. Procédé d'isolement d'un ou de plusieurs polypeptide(s) associé(s) à une séquence d'acides nucléiques cible comprenant les étapes consistant à :
(a) obtenir un échantillon comprenant une séquence d'acides nucléiques cible et un ou plusieurs polypeptide(s) associé(s) à la séquence d'acides nucléiques cible ;
(b) mettre en contact l'échantillon avec au moins une sonde oligonucléotidique comprenant une séquence complémentaire à au moins une partie de la séquence d'acides nucléiques cible et capable de s'hybrider avec celle-ci, dans lequel la sonde oligonucléotidique comprend au moins un nucléotide d'acide nucléique verrouillé (LNA) et dans lequel la sonde oligonucléotidique comprend en outre au moins un marqueur d'affinité, dans lequel le marqueur d'affinité est conjugué à la sonde oligonucléotidique par le biais d'un groupe espaceur ayant une longueur comprise entre 40 et 170 atomes ; de manière plus appropriée entre 70 et 130 atomes ; de manière plus conventionnelle entre 90 et 120 atomes ; de manière appropriée entre 100 et 110 atomes ;
(c) permettre à au moins une sonde oligonucléotidique et à la séquence d'acides nucléiques cible de s'hybrider l'une avec l'autre pour former un hybride sonde-cible ;
(d) isoler l'hybride sonde-cible de l'échantillon en immobilisant celui-ci par le biais d'une molécule qui se lie à au moins un marqueur d'affinité ; et
(e) éluer le ou les polypeptide(s) associé(s) à la séquence d'acides nucléiques cible.

2. Procédé selon la revendication 1, dans lequel la séquence d'acides nucléiques cible comprend l'ADN eucaryote, notamment l'ADN de mammifère, ou l'ADN procaryote ou l'ARN, ou les séquences d'origine virale intégrées à l'ADN de mammifère.

3. Procédé selon la revendication 2, dans lequel la séquence d'acides nucléiques cible est constituée de chromatine.

4. Procédé selon la revendication 3, dans lequel le ou les polypeptide(s) associé(s) à la séquence d'acides nucléiques cible sont des polypeptides associés à la chromatine.

5. Procédé selon l'une quelconque des revendications précédentes, dans lequel le marqueur d'affinité est choisi dans le groupe constitué par la biotine ou l'un de ses analogues, notamment la desthiobiotine ; la digoxigénine ; la fluorescéine ; le dinitrophénol et un marqueur immunohistochimique.

6. Procédé selon l'une quelconque des revendications précédentes, dans lequel l'hybride sonde-cible est immobilisé par le biais d'une molécule qui se lie à au moins un marqueur d'affinité, laquelle molécule est fixée à un substrat solide.

7. Procédé selon la revendication 6, dans lequel le substrat solide comprend une microbille, et dans lequel la microbille est capable d'être magnétiquement séparée d'une solution.

8. Procédé selon l'une quelconque des revendications précédentes, dans lequel la sonde oligonucléotidique comprend 50 % de résidus de nucléotide LNA.

9. Procédé selon l'une quelconque des revendications précédentes, dans lequel le ou les polypeptide(s) associé(s) à la séquence d'acides nucléiques cible sont exposés à des conditions provoquant la réticulation du ou des polypeptide(s) avant l'étape d'exposition de l'échantillon à la sonde oligonucléotidique, et dans lequel la réticulation est inversée avant l'étape d'élution du ou des polypeptide(s).

10. Procédé selon l'une quelconque des revendications précédentes, comprenant en outre une étape supplémentaire consistant à :
(f) analyser le ou les polypeptide(s) élué(s) par le biais d'une opération comprenant la spectrométrie de masse pour déterminer son/leur identité.

11. Sonde oligonucléotidique comprenant au moins un groupe conforme à la formule générale I décrite ci-dessous :
**A -[C]ₙ - X** **I**
dans laquelle A comprend un ou plusieurs marqueur(s) d'affinité attaché(s) à un nucléotide X par un groupe espaceur d'une longueur de n atomes, où n est compris entre 90 et 170 atomes ; A comprend un haptène ou un marqueur immunohistochimique ; et dans lequel le nucléotide X est un ribonucléotide verrouillé (LNA).

12. Sonde oligonucléotidique comprenant au moins un groupe conforme à la formule générale Il décrite ci-dessous :
**B - [C]ₙ - Y** **II**
dans laquelle B est un marqueur d'affinité attaché à une séquence oligonucléotidique Y par un groupe espaceur comprenant une chaîne linéaire de n atomes, où n est compris entre 90 et 170 atomes ; la séquence oligonucléotidique Y comprenant au moins 10 nucléotides dont au moins 10 % et jusqu'à 50 % d'entre eux sont des nucléotides d'acide nucléique verrouillé (LNA).

13. Sonde oligonucléotidique comprenant un groupe conforme à la formule générale III décrite ci-dessous : 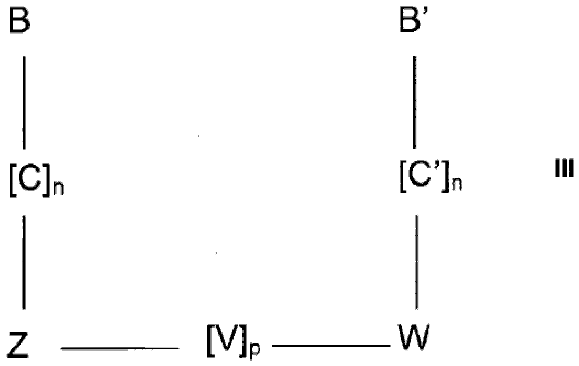 dans laquelle B et B' comprennent un marqueur d'affinité identique ou différent attaché aux nucléotides respectifs Z et W par les groupes espaceurs C et C' d'une longueur de n atomes, où n est compris entre 90 et 170 atomes ; et dans laquelle les nucléotides Z et W sont séparés par une chaîne oligonucléotidique V d'une longueur de p nucléotides, où p est compris entre 0 et 40 ; les nucléotides Z, W et V étant identiques ou différents et choisis de façon appropriée parmi un ribonucléotide, un désoxyribonucléotide, un didésoxyribonucléotide et un ribonucléotide verrouillé (LNA), et au moins l'un de Z et W est un LNA.

14. Sondes oligonucléotidiques selon les revendications 11 à 13, dans lesquelles le marqueur d'affinité est choisi dans le groupe constitué par la biotine ou l'un de ses analogues, notamment la desthiobiotine ; la digoxigénine ; la fluorescéine ; et le dinitrophénol.


### SEQUENCE LISTING

<110> Massachusetts General Hospital
   Dejardin, Jerome
   Kingston, Robert
<120> ISOLATION OF PROTEIN FACTORS THAT ASSOCIATE DIRECTLY OR INDIRECTLY WITH NUCLEIC ACIDS
<130> P20839WO/DJC
<160> 2
<170> PatentIn version 3.5
<210> 1
   <211> 25
   <212> DNA
   <213> Artificial Sequence
<220>
   <223> probe sequence
<400> 1
   ttagggttag ggttagggtt agggt 25
<210> 2
   <211> 25
   <212> DNA
   <213> Artificial Sequence
<220>
   <223> probe sequence
<400> 2
   gatgtggatg tggatgtgga tgtgg 25